# EUROPEAN PATENT SPECIFICATION

(11) **EP 3 953 358 B1**
(45) Date of publication and mention of the grant of the patent: **01.07.2026**
(21) Application number: 20786806.8
(22) Date of filing: 10.04.2020
(51) Int. Cl.: C07D 487/12, C07D 487/14, A61K 31/519, C07B 59/00, G01N 33/00, A61K 51/04, G01N 23/046, G01N 33/483

(54) **ORGANIC COMPOUNDS**
ORGANISCHE VERBINDUNGEN
COMPOSÉS ORGANIQUES

(30) Priority: 12.04.2019 US 201962833481 P
(43) Date of publication of application: 16.02.2022
(73) Proprietor: Intra-Cellular Therapies, Inc., Bedminster, NJ 07921 (US)
(72) Inventor: WENNOGLE, Lawrence P., New York, New York 10016 (US); DAVIS, Robert, New York, New York 10016 (US); LI, Peng, New York, New York 10016 (US)
(74) Representative: Carpmaels & Ransford LLP
(86) International application number: PCT/US2020/027657
(87) International publication number: WO 2020/210614

(56) References cited:
- WO-A1-2009/075784
- WO-A1-2012/171016
- WO-A1-2020/086481
- US-A1- 2013 085 123
- US-A1- 2016 038 494
- US-A1- 2017 197 974
- US-A1- 2017 291 904

## Description

### CROSS-REFERENCE TO RELATED APPLICATIONS

This application claims priority to and the benefit of U.S. Provisional Application Serial No. 62/833,481, which was filed on April 12, 2019.

### FIELD OF DISCLOSURE

The present invention relates to tracers for use in diagnostic techniques, particularly radiolabeled tracers for SPECT and positron emitter-labeled compositions for PET, detection of phosphodiesterase 1 (PDE1) activation *in vivo,* said compounds may be of use in methods for treating and/or developing novel therapies for PDE1-associated conditions, e.g., cancers and tumors, e.g., glioblastoma multiforme, and in methods of detection and treatment. The compositions of particular interest are radiolabeled compositions which selectively bind to PDE1, which is associated with conditions of interest in various tissues and organs. Positron emitter-labeled compositions targeting PDE1 would provide a basis for novel therapies for cancer, central nervous system and cardiovascular disorders.

### BACKGROUND OF THE DISCLOSURE

Gamma radiation-based imaging techniques employ tracer compounds that are introduced into the body to be imaged. The tracer compounds contain a radionuclide which directly or indirectly releases photons whose locations of origin within the body are then calculated from intercept data gathered by gamma radiation detectors. Two commonly employed gamma radiation-based imaging techniques are Positron Emission Tomography (referred to as PET) and Single Photon Emission Computed Tomography (referred to as SPECT). In PET, the radionuclide indirectly releases a pair of oppositely directed photons. The PET radionuclide emits a positron, which upon contact with an electron in its immediate vicinity triggers anti-matter annihilation of both particles which event emits the pair of photons. In SPECT, the radionuclide is a direct gamma emitter. Examples of isotopes useful in gamma radiation-based imaging include Carbon-11 (referred to as ¹¹C or C11), Fluorine-18 (referred to as ¹⁸F or F18), Technetium-99m (referred to as ⁹⁹mTc or Tc99m), Indium-111 (referred to as ¹¹¹In or In111), Iodine-123 (referred to as ¹²³I or I123), and tritium (referred to as T or ³H).

In addition to the radionuclide, the tracer compound comprises a ligand which provides an affinity of the tracer to a selected target associated with one or more tissues, organs or conditions of interest.

Type 1 Cyclic Nucleotide Phosphodiesterases (PDE1) comprise an enzyme family consisting of A, B and C isoforms, all regulated by calcium-calmodulin (Ca/CaM) to hydrolyze cyclic adenosine monophosphate (cAMP) and cyclic guanidine monophosphate (cGMP). The enzyme acts as a key regulator of these intra-cellular signal transduction messengers, particularly in excitatory cells where calcium levels rise during excitation and contraction. The enzymology of PDE1s has been well studied in vitro, and the tissue distribution of the three isoforms is well described. For other clinically interesting phosphodiesterases, these parameters have been useful in designing specific positron emitting probes that have been valuable in quantitating enzyme occupancy in humans for candidate small molecule phosphodiesterase inhibitor drugs.

There has been substantial recent interest in developing PDE1 inhibitors as therapeutic agents for human diseases, including disorders of cognition and degenerative diseases, for instance, heart failure and Parkinson's disease. The enzymology of the PDE1 family has been extensively studied and reviewed. A focal point of recent studies in the field has been calcium dependency of enzyme activity due to regulation by calcium and calmodulin, a protein. Because this enzyme is active only when intra-cellular calcium levels rise to micromolar concentrations, the contribution of PDE1 to the control over cyclic nucleotide levels in most systems is transient and almost certainly varies in different intracellular microdomains/environments. A proteolytic fragment of PDE1 devoid of the N-terminal regulatory and calmodulin-binding domain is not subject to the calcium-calmodulin requirement. This has suggested, as is the case with other PDE families, the N-terminal regulatory domain of PDE1 occludes the binding site for cyclic nucleotides, and this occlusion is released, in the case of PDE1 enzymes, by the binding of calcium-calmodulin to this regulatory domain, allowing substrate binding and enzyme hydrolytic activity. US2017/197974A1 deals with PDE1 inhibitors for the treatment of related diseases.

Glioblastoma is the most common malignant brain tumour in adults, its occurrence accounting for more than half of all primary brain tumours in a frequency of 2 to 3 per 100,000 in western developed countries. Prognosis is almost universally poor with a median survival of 6-9 months and five-year survival rates of less than 3%. Brodbelt A, et al.; (UK) National Cancer Information Network Brain Tumour Group. Glioblastoma in England: 2007-2011. Eur. J. Cancer. 2015 Mar; 51(4):533-42.

Glioblastoma is an aggressive tumour that is characterised by rapid growth and invasion into the normal brain. The efficacy of current treatments is hampered by the need to preserve normal brain function during surgery, by the tumours' intrinsic resistance to radiotherapy, and the inability of many drugs to penetrate the blood brain barrier. There has been little improvement for adult glioblastoma patients during the last three decades as successive Phase III trials of targeted agents have failed to improve survival (Touat M, et al., Glioblastoma targeted therapy: updated approaches from recent biological insights. Ann. Oncol. 2017 Jul 1;28(7):1457-1472) and temozolamide (an alkylating compound able to cross the blood brain barrier) remains one of the only effective chemotherapeutic agents available for treating glioblastoma. Even with maximal treatment of debulking surgery, radiotherapy and temozolomide, median survival is only 14.6 months. Stupp R, et al., Radiotherapy plus concomitant and adjuvant temozolomide for glioblastoma; N Engl J Med. 2005 Mar 10;352(10):987-96.

With respect to PDE1C in particular, recent evidences indicate that PDE1C is a proliferation associated gene, since it is expressed exclusively in proliferating vascular smooth muscle cells. Rybalkin SD, et al., Calmodulin-stimulated cyclic nucleotide phosphodiesterase (PDE1C) is induced in human arterial smooth muscle cells of the synthetic, proliferative phenotype. J Clin Invest 1997;100:2611-2621. In addition, there have been sporadic reports of PDE1C expression along with other PDE subtypes in experimental tumor models such as melanoma (Watanabe Y, et al., Phosphodiesterase 4 regulates the migration of B16-F10 melanoma cells. Exp Ther Med 2012;4:205-210.), neuroblastoma (Jang IS, Juhnn YS. Adaptation of cAMP signaling system in SH-SY5Y neuroblastoma cells following expression of a constitutively active G protein alpha, Q227L Gsalpha. Exp Mol Med 2001;33:37-45), and osteosarcoma (Ahlström M, et al., Cyclic nucleotide phosphodiesterases (PDEs) in human osteoblastic cells; the effect of PDE inhibition on cAMP accumulation. Cell Mol Biol Lett 2005;10:305-319).

Past studies have shown that inhibition of the PDE1 (specifically the isoform PDE1B) induces apoptosis in human leukemia cells. Jiang X, Paskind M, Weltzien R, Epstein PM. Expression and regulation of mRNA for distinct isoforms of mitogen-stimulated and leukemic human lymphocytes. Cell Biochem Biophys 1998; 28:135-60. Studies have also shown that, five out of six human glioblastoma cell lines from the National Cancer Institute show high PDE1 expression (predominantly PDE1C) and only minor PDE4 expression. Marko D, Pahlke G, Merz KH, Eisenbrand G. Cyclic 3',5'-nucleotide phosphodiesterases: potential targets for antitumor therapy. Chem Res Toxicol 2000;13:944-8. Likewise, PDE1C mRNA is overexpressed in human malignant melanoma-associated antigen (MAA) cells, and growth is inhibited by the non-selective PDE1 inhibitor vinpocetine. Zhao AZ, et al., Recent advances in the study of Ca2+/CaM-activated phosphodiesterases: expression and physiological functions. Adv Second Messenger Phosphoprotein Res 1997;31:237-51. More recent studies show that PDE1C is significantly overexpressed in 20% of glioblastoma compared to normal human brain and siRNA mediated silencing of PDE1C inhibits proliferation (45-50%) and invasion (40-60%) in patient-derived cell cultures of glioblastoma. Rowther FB, et al., Cyclic nucleotide phosphodiesterase-1C (PDE1C) drives cell proliferation, migration and invasion in glioblastoma multiforme cells in vitro. Mol Carcinog. 2016 Mar; 55(3):268-79.

Currently, there is a largely unmet need for an effective way of treating tumors of the central nervous system, such as glioblastoma multiforme. Improved therapeutic compositions and methods for the treatment and diagnosis of such conditions are urgently required.

### SUMMARY OF THE DISCLOSURE

The invention is set out in the appended claims.

The inventors have previously shown that inhibition of PDE1 activity using the presently disclosed compounds can safely maintain or restore cAMP function in a wide spectrum of pathological conditions, including models of neurodegeneration and neuroinflammation, heart failure, pulmonary hypertension and peripheral inflammation and in humans with certain diseases. More recently, the inventors have shown that PDE1 inhibitors reduce cellular migration of microglia and monocytes. Recent evidence indicates that PDE1, particularly the PDE1C isoform, is over-expressed in experimental tumor models such as melanoma, neuroblastoma, and osteosarcoma. In addition, focal genomic over representation of PDE1C in Glioblastoma Multiforme (GBM) cells has been demonstrated. Genomic gain of PDE1C is associated with increased expression in GBM-derived cell cultures and is essential for driving cell proliferation, migration and invasion in cancer cells.

Many types of cancer cells over express PDE1 activity, which is identified through various biomarkers, such as increased RNA expression, DNA copy number, PDE1 binding (PET or radio-isotope retention of PDE1 inhibitor molecules) or enzymatic activity. These cancer cells also exhibit low levels of cAMP, allowing for more rapid cancer cell invasion and proliferation, which can be increased by PDE1 inhibitors. Such characteristics can be treated with PDE-1 inhibitors alone or in combination with chemotherapeutics, gene therapeutics and/or immunologic approaches. In addition, inhibiting PDE1 provokes apoptotic cell death, prevents migration, limits metastasis, and reduces inflammation. In this way, PDE1 inhibitors are synergistic with chemotherapeutics and immunologic approaches.

It is also known that in many cell types and in certain cancers, e.g., glioblastoma tumors, intracellular calcium levels are elevated. Elevated calcium levels activate calcium-activated proteases such as calpain. Calpain is known to cleave off the N-terminal regulatory domain of PDE1, providing an active catalytic domain whose activity is no longer calcium/calmodulin sensitive. Activation of PDE1 results in a reduction of intracellular cyclic nucleotide concentrations and may potentiate pathways involved in cancer development.

Since many types of cancers display elevated amounts of PDE1, it is believed that radiolabeled PDE1 inhibitors would be useful in the diagnosis and subsequent treatment of such cancers or other conditions resulting in heightened levels of PDE1. However, efforts in creating a radiolabeled compound to selectively detect PDE1 have been relatively unsuccessful. The present inventors have created novel compounds and radioligands which are effective and selective PDE1 radiotracers.

Therefore, in various embodiments, the present invention provides novel compounds according to Formula Ia and/or Formula II, as defined in claim 1.
In one aspect, the present invention provides a radiolabeled compound selected from:
(A) Formula Ia: 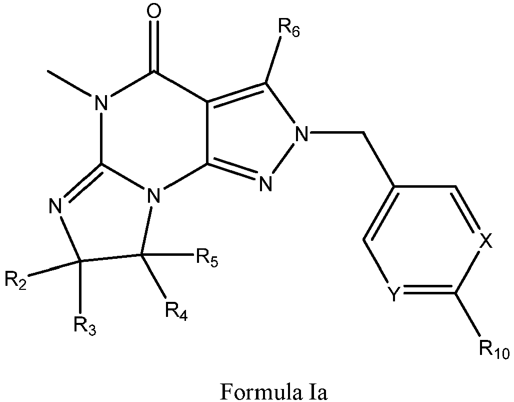 wherein
   (i) R₂ and R₅ are independently H or hydroxy and R₃ and R₄ together form a tri- or tetra-methylene bridge [pref. with the carbons carrying R₃ and R₄ having the R and S configuration respectively]; or R₂ and R₃ are each methyl and R₄ and R₅ are each H; or R₂, R₄ and R₅ are H and R₃ is isopropyl [pref. the carbon carrying R₃ having the R configuration];
   (ii) R₆ is (optionally halo-substituted) phenylamino, (optionally halo-substituted) benzylamino, C₁₋₄alkyl, or C₁₋₄alkyl sulfide; for example, phenylamino or 4-fluorophenylamino;
   (iii) R₁₀ is 1-methylpyrrolidin-2-yl substituted at one or more positions with tritium (T) in place of H; and
   (iv) X and Y are independently C or N,
   in free, pharmaceutically acceptable salt form, including its enantiomers, diastereoisomers and racemates; and
(B) Formula II: 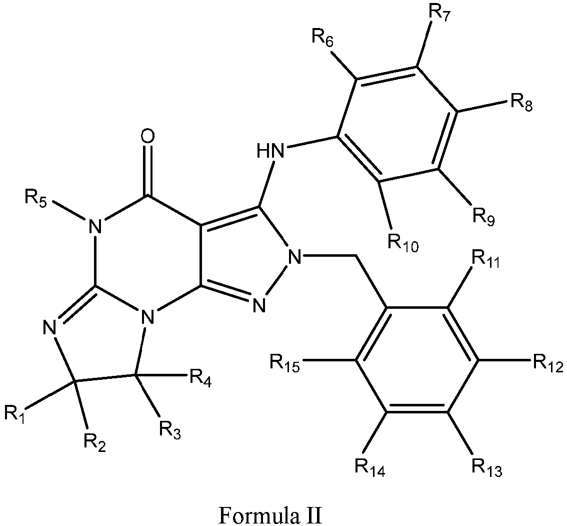 wherein
   R₁, R₂ and R₅ are independently selected from optionally T-substituted C₁₋₄alkyl, H or T (tritium, ³H);
   R₃, R₄, R₁₁, R₁₂, R₁₄ and R₁₅ are independently selected from H or T (tritium, ³H);
   R₆, R₇, R₈, R₉ and R₁₀ are independently selected from halogen (e.g., F), H or T;
   R₁₃ is 1-methylpyrrolidin-2-yl substituted at one or more positions with tritium (T) in place of H; in free, pharmaceutically acceptable salt form, including its enantiomers, diastereoisomers and racemates.

In a further aspect, the present invention provides a compound for use in a method of mapping functional PDE1 activity in a tissue and/or organ of interest using positron emission tomography which comprises administering an effective amount of a PDE1 radiotracer compound according to Formula Ia *et seq.* or II *et seq.* to the tissue and/or organ; allowing a period of time sufficient for the PDE1 radiotracer to effectively associate with PDE1 in the tissues and/or organ of interest; and analyzing the tissues and organs of interest using positron emission tomography.

In another aspect, the present invention provides a compound for use in a method [Method 3] of diagnosing a PDE1-mediated disease, disorder or condition characterized by up-regulation of PDE1 expression in a subject, wherein the PDE1-mediated disease, disorder or condition is a cardiovascular-related disorder, a neurodegenerative disease, a cancer or a tumor, comprising obtaining a first tissue sample from a patient suspected of having the PDE1-mediated disease, disorder or condition or at risk for the PDE1-mediated disease, disorder or condition; contacting the first tissue sample with an effective amount of a compound according to Formula Ia *et seq.* or II *et seq.;* imaging the first tissue sample with a positron emission tomography device; and comparing the results of the previous step to a second tissue sample in which PDE1 expression is not up-regulated. In some embodiments, the method further comprises quantitating PDE1 expression *ex vivo* via biopsies taken from patients suffering from glioblastoma.

### BRIEF DESCRIPTION OF THE DRAWINGS

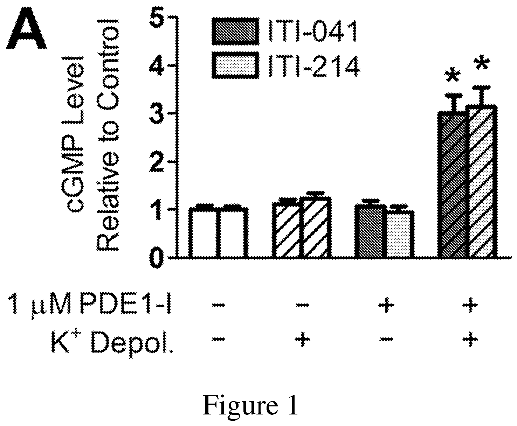

Figure 1 illustrates the effect of PDE1 inhibition on striatal cGMP levels. Striatal slices were preincubated with either vehicle or compound, followed by a 1-minute stimulation with high K+ or vehicle. cGMP levels were normalized to baseline levels for comparison. As described, the baseline cGMP levels did not vary greatly between preparations. PDE1 inhibition by 1 µM ITI-041 or 1 µM ITI-214 in the presence of K+ depolarization (striped bars). Depolarization leads to increased intracellular calcium levels and activation of PDE1 enzymes, which when inhibited by PDE1 inhibitors leads to increased cyclic nucleotide levels.

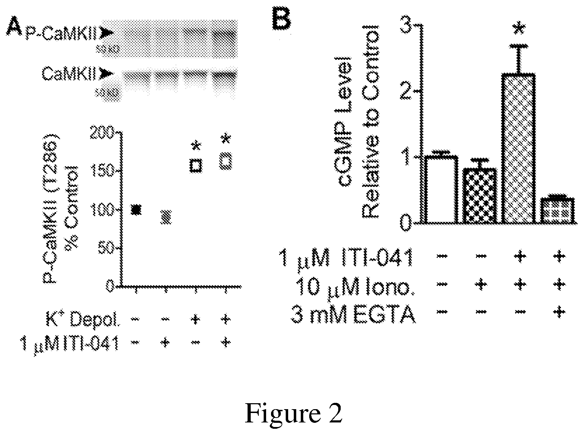

Figure 2 illustrates that increased Ca2+ is sufficient to unmask the active site of PDE1 and allow PDE1 inhibitor binding to the active site therefore increasing cGMP. Increased intracellular calcium is sufficient to increase cGMP levels in the presence of PDE1 inhibitor. Part A shows phosphorylation of CaMKII at T286 and total CaMKII measured using a standard Western blotting method (top) and quantitation (bottom). Each treatment with K+ depolarization has significantly higher labeling of phosphorylated CaMKII than control. Part B shows the effects of ionomycin on striatal cGMP. cGMP levels in the presence of 10 µM ionomycin and 1 µM ITI-041, a PDE1 inhibitor, are significantly higher than either control or ionomycin alone and EGTA, n = 8.

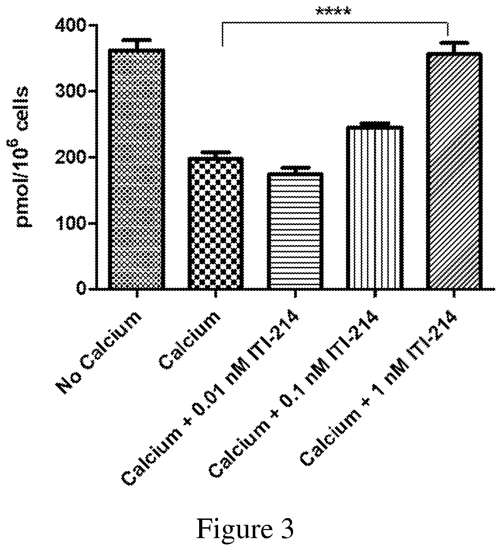

Figure 3 illustrates the dose-response of a PDE1 inhibitor to reverse cAMP downregulated by calcium in 1321N1 human astrocytoma cells. Inhibition of PDE1 is evident even at sub-nanomolar concentrations of the PDE1 inhibitor in this cellular system.

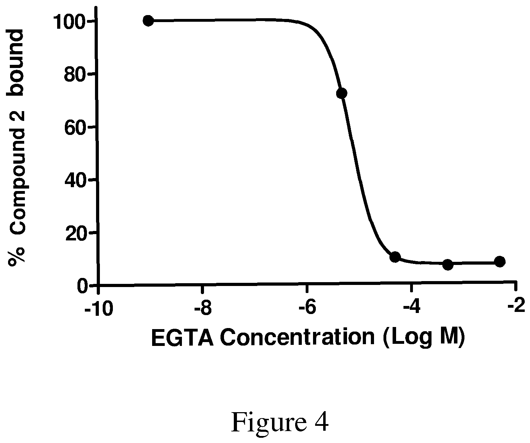

Figure 4 illustrates Ca²⁺-dependent binding of Compound 2, as defined in Example 1, to mouse cortex. Binding of the radioligand is prevented in the presence of EGTA, a specific chelator of calcium

### DETAILED DESCRIPTION OF THE DISCLOSURE

### Compounds for use in the methods of the disclosure

Any reference in the description to a method of treatment of the human (or animal) body by therapy (or for diagnosis), to the use in a treatment, or to a therapy is to be interpreted as reference to compounds, pharmaceutical compositions or combinations of the present invention for use in such methods, treatments or therapies.

In one embodiment, the PDE1 inhibitors for use in the methods of treatment, diagnosis, detection, and prophylaxis described herein are selective PDE1 inhibitors.

### PDE1 Inhibitors

In one embodiment the invention provides for PDE1 inhibitors, optionally for use in the methods of treatment diagnosis, detection, and prophylaxis described herein, are compounds of Formula Ia: 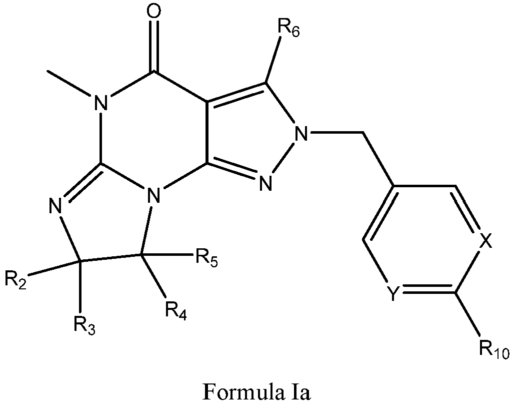 wherein
(i) R₂ and R₅ are independently H or hydroxy and R₃ and R₄ together form a tri- or tetra-methylene bridge [pref. with the carbons carrying R₃ and R₄ having the R and S configuration respectively]; or R₂ and R₃ are each methyl and R₄ and R₅ are each H; or R₂, R₄ and R₅ are H and R₃ is isopropyl [pref. the carbon carrying R₃ having the R configuration];
(ii) R₆ is (optionally halo-substituted) phenylamino, (optionally halo-substituted) benzylamino, C₁₋₄alkyl, or C₁₋₄alkyl sulfide; for example, phenylamino or 4-fluorophenylamino;
(iii) R₁₀ is 1-methylpyrrolidin-2-yl substituted at one or more positions with tritium (T) in place of H; and
(iv) X and Y are independently C or N,
in free, pharmaceutically acceptable salt, or prodrug form, including its enantiomers, diastereoisomers and racemates.

In further embodiments, the invention provides compounds of Formula I as described in the following formulae:
1.1 The Compound of Formula Ia, comprising at least one T substituent in place of H.
1.2 The compound of Formula Ia or 1.1, wherein R₂ and R₅ are independently H, T or hydroxy and R₃ and R₄ together form a tri- or tetra-methylene bridge [pref. with the carbons carrying R₃ and R₄ having the R and S configuration respectively].
1.3 The compound of Formula Ia or 1.1-1.2, wherein R₂ and R₃ are each methyl and R₄ and R₅ are each H or T.
1.4 The compound of Formula Ia or 1.1-1.3, wherein R₂, R₄ and R₅ are H or T and R₃ is isopropyl [pref. the carbon carrying R₃ having the R configuration].
1.5 The compound of Formula Ia or 1.1-1.4, wherein R₆ is phenylamino.
1.6 The compound of Formula Ia or 1.1-1.5, wherein R₆ is optionally halo-substituted phenylamino.
1.7 The compound of Formula Ia or 1.1-1.6, wherein R₆ is halo-substituted phenylamino.
1.8 The compound of Formula Ia or 1.1-1.7, wherein R₆ is optionally fluoro substituted phenylamino.
1.9 The compound of Formula Ia or 1.1-1.8, wherein R₆ is 4-fluorophenylamino.
1.19 The compound of Formula Ia or 1.1-1.10, wherein R₁₀ is pyrrolidyl substituted with tritiated methyl.
1.20 The compound of Formula Ia or 1.1-1.10, wherein R₁₀ is 1-methylpyrrolidin-2-yl, wherein the methyl substituent is fully or partially tritiated.
1.21 The compound of Formula Ia or 1.1-1.10, wherein R₁₀ is 1-methylpyrrolidin-2-yl, wherein the methyl substituent is fully tritiated.
1.22 The compound of Formula Ia or 1.1-1.21, wherein X and Y are C.
1.23 The compound of Formula Ia or 1.1-1.22, wherein R₂ and R₃ are each methyl and R₄ and R₅ are each H or T; R₆ is optionally halo-substituted phenylamino; R₁₀ is 1-methylpyrrolidin-2-yl substituted at one or more positions with tritium (T) in place of H, and X and Y are C.
1.25 The compound of Formula Ia, wherein the compound is: 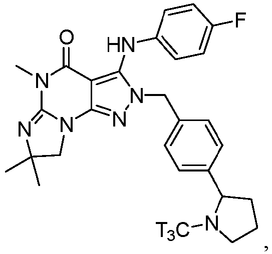 in free, pharmaceutically acceptable salt or prodrug form.

In another embodiment the invention provides for PDE1 inhibitors, potentially for use in the methods of treatment and prophylaxis described herein, are compounds of Formula II: 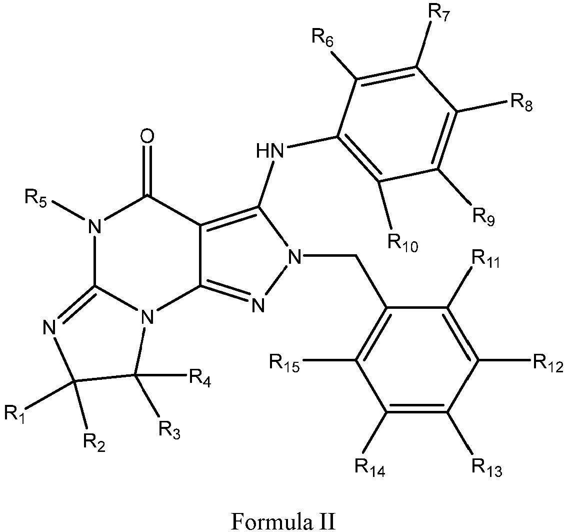 wherein
R₁, R₂ and R₅ are independently selected from optionally T-substituted C₁₋₄alkyl, H or T (tritium, ³H);
R₃, R₄, R₁₁, R₁₂, R₁₄ and R₁₅ are independently selected from H or T;
R₆, R₇, R₈, R₉ and R₁₀ are independently selected from halogen (e.g., F), H or T;
R₁₃ is 1-methylpyrrolidin-2-yl substituted at one or more positions with tritium (T) in place of H;
in free, pharmaceutically acceptable salt, or prodrug form, including its enantiomers, diastereoisomers and racemates.

In further embodiments, the invention provides compounds of Formula I as described in the following formulae:
2.1 The compound of Formula II, wherein R₁, R₂ and R₅ are independently optionally T-substituted C₁₋₄alkyl.
2.2 The compound of Formula II or 2.1, wherein R₁, R₂ and R₅ are independently T-substituted C₁₋₄alkyl.
2.3 The compound of Formula II or any of 2.1-2.2, wherein R₁, R₂ and R₅ are each optionally T-substituted methyl.
2.4 The compound of Formula II or any of 2.1-2.3, wherein R₃, R₄, R₁₁, R₁₂, R₁₄ and R₁₅ are H.
2.11 The Compound of Formula II or any of 2.1-2.7, wherein R₁₃ is 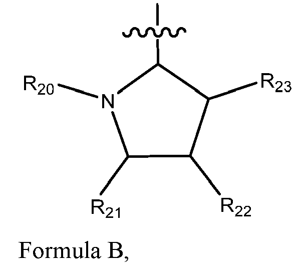 wherein
   R₂₁, R₂₂ and R₂₃ are independently selected from H or T; and
   R₂₀ is T-substituted C₁₋₄alkyl.
2.12 The Compound of Formula II or any of 2.1-2.7, wherein R₁₃ is 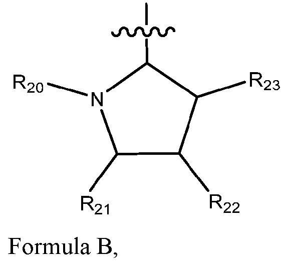 wherein
   R₂₁, R₂₂ and R₂₃ are H; and
   R₂₀ is T-substituted methyl.
2.13 The Compound of 2.12, wherein R₂₀ is T₃C.
2.15 The compound of Formula II, wherein the compound is: 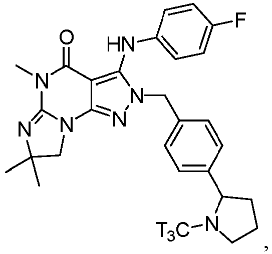 in free, pharmaceutically acceptable salt or prodrug form.

In some embodiments the invention provides for a PDE1 inhibitor, potentially for use in the methods of treatment and prophylaxis described herein, wherein the inhibitor is a compound according to the following: 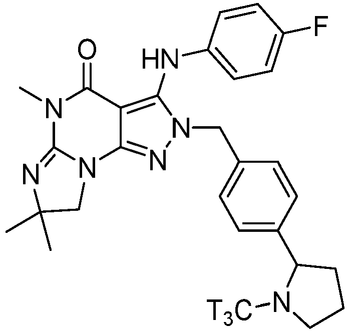 in free or pharmaceutically acceptable salt form.

In one embodiment, selective PDE1 inhibitors of any of the preceding formulae (e.g., Formula Ia or II) are compounds that inhibit phosphodiesterase-mediated (e.g., PDE1-mediated, especially PDE1B-mediated) hydrolysis of cGMP and by inference cAMP, e.g., the preferred compounds have an IC50 of less than 1µM, preferably less than 500 nM, preferably less than 50 nM, and preferably less than 5nM in an immobilized-metal affinity particle reagent PDE assay, in free or salt form.

Further examples of PDE1 inhibitors which can be proton or radiolabeled for use in the methods and treatments discussed herein can be found in International Publication WO2006133261A2; U.S. Patent 8,273,750; U.S. Patent 9,000,001; U.S. Patent 9,624,230; International Publication WO2009075784A1; U.S. Patent 8,273,751; U.S. Patent 8,829,008; U.S. Patent 9,403,836; International Publication WO2014151409A1, U.S. Patent 9,073,936; U.S. Patent 9,598,426; U.S. Patent 9,556,186; U.S. Publication 2017/0231994A1, International Publication WO2016022893A1, and U.S. Publication 2017/0226117A1.

Still further examples of PDE1 inhibitors suitable which can be radiolabeled, e.g. tritiated, for use in the methods and treatments discussed herein can be found in International Publication WO2018007249A1; U.S. Publication 2018/0000786; International Publication WO2015118097A1; U.S. Patent 9,718,832; International Publication WO2015091805A1; U.S. Patent 9,701,665; U.S. Publication 2015/0175584A1; U.S. Publication 2017/0267664A1; International Publication WO2016055618A1; U.S. Publication 2017/0298072A1; International Publication WO2016170064A1; U.S. Publication 2016/0311831A1; International Publication WO2015150254A1; U.S. Publication 2017/0022186A1; International Publication WO2016174188A1; U.S. Publication 2016/0318939A1; U.S. Publication 2017/0291903A1; International Publication WO2018073251A1; International Publication WO2017178350A1; and U.S. Publication 2017/0291901A1.

Additional examples of PDE1 inhibitors and compounds suitable for use as PDE1 radiotracers are disclosed in International Publication WO2011043816A1 and U.S. Patent 8,858,911.

If not otherwise specified or clear from context, the following terms herein have the following meanings:
**(a)** "Selective PDE1 inhibitor" as used herein refers to a PDE1 inhibitor with at least 100-fold selectivity for PDE1 inhibition over inhibition of any other PDE isoform families covering PDE2 to PDE11.
**(b)** "Alkyl" as used herein is a saturated or unsaturated hydrocarbon moiety, preferably saturated, preferably having one to six carbon atoms, which may be linear or branched, and may be optionally mono-, di- or tri- substituted, e.g., with halogen (e.g., chloro or fluoro), hydroxy, or carboxy.
**(c)** "Cycloalkyl" as used herein is a saturated or unsaturated nonaromatic hydrocarbon moiety, preferably saturated, preferably comprising three to nine carbon atoms, at least some of which form a nonaromatic mono- or bicyclic, or bridged cyclic structure, and which may be optionally substituted, e.g., with halogen (e.g., chloro or fluoro), hydroxy, or carboxy. Wherein the cycloalkyl optionally contains one or more atoms selected from N and O and/or S, said cycloalkyl may also be a heterocycloalkyl.
**(d)** "Heterocycloalkyl" is, unless otherwise indicated, saturated or unsaturated nonaromatic hydrocarbon moiety, preferably saturated, preferably comprising three to nine carbon atoms, at least some of which form a nonaromatic mono- or bicyclic, or bridged cyclic structure, wherein at least one carbon atom is replaced with N, O or S, which heterocycloalkyl may be optionally substituted, e.g., with halogen (e.g., chloro or fluoro), hydroxy, or carboxy.
**(e)** "Aryl" as used herein is a mono or bicyclic aromatic hydrocarbon, preferably phenyl, optionally substituted, e.g., with alkyl (e.g., methyl), halogen (e.g., chloro or fluoro), haloalkyl (e.g., trifluoromethyl), hydroxy, carboxy, or an additional aryl or heteroaryl (e.g., biphenyl or pyridylphenyl).
**(f)** "Heteroaryl" as used herein is an aromatic moiety wherein one or more of the atoms making up the aromatic ring is sulfur or nitrogen rather than carbon, e.g., pyridyl or thiadiazolyl, which may be optionally substituted, e.g., with alkyl, halogen, haloalkyl, hydroxy or carboxy."
**(g)** "Substituted with T (tritium, ³H) or D (deuterium, ²H) at one or more positions in place of H" or similar language means that the frequency of the particular hydrogen isotope (tritium or deuterium) at that position is higher than the frequency of H (protium) at that position.

Compounds of the Disclosure, e.g., PDE1 inhibitors as described herein, may exist in free or salt form, e.g., as acid addition salts. In this specification unless otherwise indicated, language such as "Compounds of the Disclosure" is to be understood as embracing the compounds in any form, for example free or acid addition salt form, or where the compounds contain acidic substituents, in base addition salt form. The Compounds of the Disclosure are intended for use as pharmaceuticals, therefore pharmaceutically acceptable salts are preferred. Salts which are unsuitable for pharmaceutical uses may be useful, for example, for the isolation or purification of free Compounds of the Disclosure or their pharmaceutically acceptable salts, are therefore also included.

Compounds of the Disclosure may in some cases also exist in prodrug form. A prodrug form is compound which converts in the body to a Compound of the Disclosure. For example, when the Compounds of the Disclosure contain hydroxy or carboxy substituents, these substituents may form physiologically hydrolysable and acceptable esters. As used herein, "physiologically hydrolysable and acceptable ester" means esters of Compounds of the Disclosure which are hydrolysable under physiological conditions to yield acids (in the case of Compounds of the Disclosure which have hydroxy substituents) or alcohols (in the case of Compounds of the Disclosure which have carboxy substituents) which are themselves physiologically tolerable at doses to be administered. Therefore, wherein the Compound of the Disclosure contains a hydroxy group, for example, Compound-OH, the acyl ester prodrug of such compound, i.e., Compound-O-C(O)-C1-4alkyl, can hydrolyze in the body to form physiologically hydrolysable alcohol (Compound-OH) on the one hand and acid on the other (e.g., HOC(O)-C1-4alkyl). Alternatively, wherein the Compound of the Disclosure contains a carboxylic acid, for example, Compound-C(O)OH, the acid ester prodrug of such compound, Compound-C(O)O-C1-4alkyl can hydrolyze to form Compound-C(O)OH and HO-C1-4alkyl. As will be appreciated the term thus embraces conventional pharmaceutical prodrug forms.

In another embodiment, the disclosure further provides a pharmaceutical composition comprising a PDE1 inhibitor in combination with an antitumor agent, each in free or pharmaceutically acceptable salt form, in admixture with a pharmaceutically acceptable carrier. The term "combination," as used herein, embraces simultaneous, sequential, or contemporaneous administration of the PDE1 inhibitor and the antitumor agent. In another embodiment, the disclosure provides a pharmaceutical composition containing such a compound. In some embodiments, the combination of the PDE1 inhibitor and the antitumor agent allows the antitumor agent to be administered in a dosage lower than would be effective if administered as sole monotherapy.

### Methods of Using Compounds of the Disclosure

In another embodiment, the present invention provides a compound for use in a method [Method 1] of mapping levels of activated PDE1 activity in a tissue and/or organ of interest using positron emission tomography, comprising:
(a) administering an effective amount of a PDE1 tracer compound according to Formula Ia *et seq.* or II *et seq.* to the tissue and/or organ (optionally by administering the PDE1 tracer compound to a subject *in vivo* or by administering the PDE1 tracer compound to a tissue sample *ex vivo*);
(b) allowing a period of time sufficient for the PDE1 tracer to effectively associate with PDE1 in the tissues and/or organs of interest; and
(c) analyzing the tissues and/or organs of interest using positron emission tomography.

In further embodiments, the invention provides Method 1 as described in the following Methods:
1.2 Any of the preceding Methods, wherein the compound is 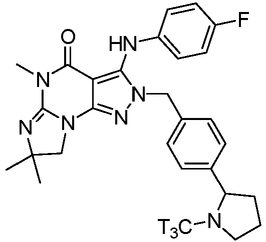 in free or pharmaceutically acceptable salt form.
1.3 Any of the preceding Methods, wherein the period of time sufficient for the tracer to effectively associate with PDE1 in the tissues and/or organs of interest is a period of about 1 minute, about 5 minutes, about 10 minutes, about 15 minutes, about 20 minutes, about 25 minutes, about 30 minutes, about 35 minutes, about 40 minutes, about 45 minutes, about 50 minutes, about 55 minutes, about 60 minutes, about 65 minutes, about 70 minutes, about 75 minutes, about 80 minutes, about 85 minutes, about 90 minutes, about 95 minutes, about 100 minutes, about 105 minutes, about 110 minutes, about 115 minutes, or about 120 minutes.
1.4 Any of the preceding Methods, wherein the period of time sufficient for the tracer to effectively associate with PDE1 in the tissues and/or organs of interest is a period of about 60 minutes.
1.5 Any of the preceding methods, wherein the mapping is selective and reversible.
1.6 Any of the preceding methods, wherein the binding of the PDE1 tracer compound to activated PDE1 in the tissues or organs is detected *in vitro.*
1.7 Any of the preceding methods, wherein the detecting binding of the PDE1 tracer compound to activated PDE1 in the tissues or organs is detected *in vivo.*
1.8 Any of the preceding methods, wherein the compound according to Formula Ia or II is administered intravenously.
1.9 Any of the preceding methods, wherein the positron emission tomography is positron emission tomography/computed tomography or single photon emission computed tomography/computed tomography.
1.10 Any of the preceding methods, wherein the tissue is from a patient suffering from a PDE1-mediated disease, disorder or condition.
1.11 Method 1.10, wherein the PDE1-mediated disease, disorder or condition is a cardiovascular-related disorder, a neurodegenerative disease, a cancer or a tumor.
1.12 Method 1.10, wherein the PDE1-mediated disease, disorder or condition is:
   a. a tumor selected from one or more of acoustic neuroma, astrocytoma, chordoma, CNS lymphoma, craniopharyngioma, gliomas (e.g., Brain stem glioma, ependymoma, mixed glioma, optic nerve glioma), subependymoma, medulloblastoma, meningioma, metastatic brain tumors, oligodendroglioma, pituitary tumors, primitive neuroectodermal (PNET), schwannoma, adenomas (e.g., basophilic adenoma, eosinophilic adenoma, chromophobe adenoma, parathyroid adenoma, islet adenoma, fibroadenoma), fibroids (fibrous histiocytoma), fibromas, hemangiomas, lipomas (e.g., angiolipoma, myelolipoma, fibrolipoma, spindle cell lipoma, hibernoma, atypical lipoma), myxoma, osteoma, preleukemias, rhadomyoma, papilloma, seborrheic keratosis, skin adnexal tumors, hepatic adenomas, renal tubular adenoma, bile duct adenoma, transitional cell papilloma, hydatidiform moles, ganglioneuroma, meningoma, neurilemmoma, neurofibroma, C cell hyperplasia, pheochromocytoma, insulinoma, gastrinoma, carcinoids, chemodectoma, paraganglioma, nevus, actinic keratosis, cervical dysplasia, metaplasia (e.g., metaplasia of the lung), leukoplakia, hemangioma, lymphangioma, carcinoma (e.g., squamous cell carcinoma, epidermoid carcinoma, adenocarcinoma, hepatoma, hepatocellular carcinoma, renal cell carcinoma, cholangiocarcinoma, transitional cell carcinoma, embryonal cell carcinoma, parathyroid carcinoma, medullary carcinoma of thyroid, bronchial carcinoid, oat cell carcinoma, islet cell carcinoma, malignant carcinoid,), sarcoma (e.g., fibrosarcoma, myxosarcoma, liposarcoma, chondrosarcoma, osteosarcoma, malignant fibrous histiocytoma, hemangiosarcoma, angiosarcoma, lymphangiosarcoma, leiomyosarcoma, rhabdomyosarcoma, neurofibrosarcoma), blastoma (e.g., medulloblastoma and glioblastoma, types of brain tumor, retinoblastoma, a tumor in the retina of the eye, osteoblastoma, bone tumors, neuroblastoma), germ cell tumor, mesothelioma, malignant skin adnexal tumors, hypernephroma, seminoma, glioma, malignant meningioma, malignant shwannoma, malignant pheochromocytoma, malignant paraganglioma, melanoma, mercell cell neoplasm, cystosarcoma phylloides, or Wilms tumor;
   b. a cancer selected from leukemia (i.e., lymphoctic leukemia or a myelogenous leukemia);
   c. a cardiovascular disorder selected from angina, stroke, renal failure, essential hypertension, pulmonary hypertension, secondary hypertension, isolated systolic hypertension, hypertension associated with diabetes, hypertension associated with atherosclerosis, renovascular hypertension, congestive heart failure, myocardial infarction, angina, stroke and renal failure, hypertension, an inflammatory disease or disorder, Duchenne muscular dystrophy, Becker muscular dystrophy, limb-girdle muscular dystrophy, myotonic dystrophy, and Emery-Dreifuss muscular dystrophy and an connective tissue disease or disorder (e.g., Marfan Syndrome);
   d. a neurodegenerative disease selected from Parkinson's disease, restless leg, tremors, dyskinesias, Huntington's disease, Alzheimer's disease, and drug-induced movement disorders; Mental disorders, including depression, attention deficit disorder, attention deficit hyperactivity disorder, bipolar illness, anxiety, sleep disorders, e.g., narcolepsy, cognitive impairment, dementia, Tourette's syndrome, autism, fragile X syndrome, psychostimulant withdrawal, and drug addiction;
   e. respiratory and inflammatory disorders selected from asthma, chronic obstructive pulmonary disease, and allergic rhinitis, as well as autoimmune and inflammatory diseases;
   f. any disease or condition characterized by low levels of cAMP and/or cGMP (or inhibition of cAMP and/or cGMP signaling pathways) in cells expressing PDE1; and/or
   g. any disease or condition characterized by reduced dopamine D1 receptor signaling activity.
1.13 Method 1.10, wherein the condition is a glioma, osteosarcoma, melanoma, leukemia, or neuroblastoma.
1.14 Method 1.10, wherein the PDE1-mediated disease, disorder or condition is a glioma (e.g., ependymoma, astrocytoma, oligodendrogliomas, brain stem glioma, optic nerve glioma, or mixed gliomas, e.g., oligoastrocytomas).
1.15 Method 1.10, wherein the glioma is an astrocytoma (e.g., glioblastoma multiforme).
1.16 Any of Methods 1.10-1.15, wherein the condition is glioblastoma multiforme.

In another embodiment, the invention provides a PET-scan image produced by any of Method 1, et seq..

Method 2 is included for reference.
In another embodiment, the present disclosure provides a method of treatment for a PDE1-mediated disease, disorder or condition [Method 2] to a subject in need thereof comprising:
a) administering an effective amount of a compound according to Formula Ia *et seq.* and/or II *et seq.* to the subject, wherein the compound according to Formula Ia *et seq.* and/or II *et seq* is radiolabeled, e.g., substituted with T (tritium, ³H) at one or more positions in place of H;
b) imaging the subject with a positron emission tomography device;
c) administering a PDE1 inhibitor to the subject, wherein the PDE1 inhibitor is not substituted with T (tritium, ³H) at one or more positions in place of H;
d) imaging the subject with a positron emission tomography device;
e) comparing the data thus obtained, and
f) assessing the delivery of the PDE1 inhibitor to a tissue of interest in the PDE1-mediated condition by detecting displacement of the compound according to Formula Ia *et seq.* and/or II *et seq.*

In further embodiments, the disclosure provides Method 2 as described in the following Methods:
2.1 Method 2, wherein at least one position on the compound according to Formula Ia *et seq.* and/or II *et seq.* of step a) is substituted with tritium (T) in place of H.
2.2 Any of the preceding Methods, wherein the compound of step a) is 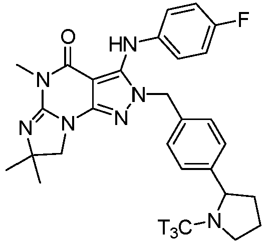 in free or pharmaceutically acceptable salt form.
2.3 Any of the preceding methods, wherein the positron emission tomography is positron emission tomography/computed tomography or single photon emission computed tomography/computed tomography.
2.4 Any of the preceding methods, wherein the tissue is from a patient suffering from a PDEI1-mediated disease, disorder or condition.
2.5 Any of the preceding methods, wherein the PDE1-mediated disease, disorder or condition is a cardiovascular-related disorder, a neurodegenerative disease, a cancer or a tumor.
2.6 Any of the preceding methods, wherein the PDE1-mediated disease, disorder or condition is:
   a. a tumor selected from one or more of acoustic neuroma, astrocytoma, chordoma, CNS lymphoma, craniopharyngioma, gliomas (e.g., Brain stem glioma, ependymoma, mixed glioma, optic nerve glioma), subependymoma, medulloblastoma, meningioma, metastatic brain tumors, oligodendroglioma, pituitary tumors, primitive neuroectodermal (PNET), schwannoma, adenomas (e.g., basophilic adenoma, eosinophilic adenoma, chromophobe adenoma, parathyroid adenoma, islet adenoma, fibroadenoma), fibroids (fibrous histiocytoma), fibromas, hemangiomas, lipomas (e.g., angiolipoma, myelolipoma, fibrolipoma, spindle cell lipoma, hibernoma, atypical lipoma), myxoma, osteoma, preleukemias, rhadomyoma, papilloma, seborrheic keratosis, skin adnexal tumors, hepatic adenomas, renal tubular adenoma, bile duct adenoma, transitional cell papilloma, hydatidiform moles, ganglioneuroma, meningoma, neurilemmoma, neurofibroma, C cell hyperplasia, pheochromocytoma, insulinoma, gastrinoma, carcinoids, chemodectoma, paraganglioma, nevus, actinic keratosis, cervical dysplasia, metaplasia (e.g., metaplasia of the lung), leukoplakia, hemangioma, lymphangioma, carcinoma (e.g., squamous cell carcinoma, epidermoid carcinoma, adenocarcinoma, hepatoma, hepatocellular carcinoma, renal cell carcinoma, cholangiocarcinoma, transitional cell carcinoma, embryonal cell carcinoma, parathyroid carcinoma, medullary carcinoma of thyroid, bronchial carcinoid, oat cell carcinoma, islet cell carcinoma, malignant carcinoid,), sarcoma (e.g., fibrosarcoma, myxosarcoma, liposarcoma, chondrosarcoma, osteosarcoma, malignant fibrous histiocytoma, hemangiosarcoma, angiosarcoma, lymphangiosarcoma, leiomyosarcoma, rhabdomyosarcoma, neurofibrosarcoma), blastoma (e.g., medulloblastoma and glioblastoma, types of brain tumor, retinoblastoma, a tumor in the retina of the eye, osteoblastoma, bone tumors, neuroblastoma), germ cell tumor, mesothelioma, malignant skin adnexal tumors, hypernephroma, seminoma, glioma, malignant meningioma, malignant shwannoma, malignant pheochromocytoma, malignant paraganglioma, melanoma, mercell cell neoplasm, cystosarcoma phylloides, or Wilms tumor;
   b. a cancer selected from leukemia (i.e., lymphoctic leukemia or a myelogenous leukemia);
   c. a cardiovascular disorder selected from angina, stroke, renal failure, essential hypertension, pulmonary hypertension, secondary hypertension, isolated systolic hypertension, hypertension associated with diabetes, hypertension associated with atherosclerosis, renovascular hypertension, congestive heart failure, myocardial infarction, angina, stroke and renal failure, hypertension, an inflammatory disease or disorder, Duchenne muscular dystrophy, Becker muscular dystrophy, limb-girdle muscular dystrophy, myotonic dystrophy, and Emery-Dreifuss muscular dystrophy and an connective tissue disease or disorder (e.g., Marfan Syndrome);
   d. a neurodegenerative disease selected from Parkinson's disease, restless leg, tremors, dyskinesias, Huntington's disease, Alzheimer's disease, and drug-induced movement disorders; Mental disorders, including depression, attention deficit disorder, attention deficit hyperactivity disorder, bipolar illness, anxiety, sleep disorders, e.g., narcolepsy, cognitive impairment, dementia, Tourette's syndrome, autism, fragile X syndrome, psychostimulant withdrawal, and drug addiction;
   e. respiratory and inflammatory disorders selected from asthma, chronic obstructive pulmonary disease, and allergic rhinitis, as well as autoimmune and inflammatory diseases;
   f. any disease or condition characterized by low levels of cAMP and/or cGMP (or inhibition of cAMP and/or cGMP signaling pathways) in cells expressing PDE1; and/or
   g. any disease or condition characterized by reduced dopamine D1 receptor signaling activity.
2.7 Any of the preceding Methods, wherein the condition is a glioma, osteosarcoma, melanoma, leukemia, or neuroblastoma.
2.8 Any of the preceding Methods, wherein the PDE1-mediated disease, disorder or condition is a glioma (e.g., ependymoma, astrocytoma, oligodendrogliomas, brain stem glioma, optic nerve glioma, or mixed gliomas, e.g., oligoastrocytomas).
2.9 Any of the preceding Methods, wherein the glioma is an astrocytoma (e.g., glioblastoma multiforme).
2.10 Any of the preceding Methods, wherein the condition is glioblastoma multiforme.
2.11 Any of the preceding Methods, wherein the PDE1 inhibitor administered in step c) is selected from the following: 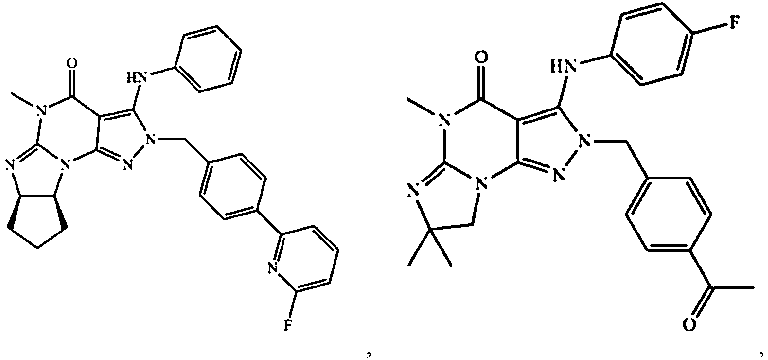 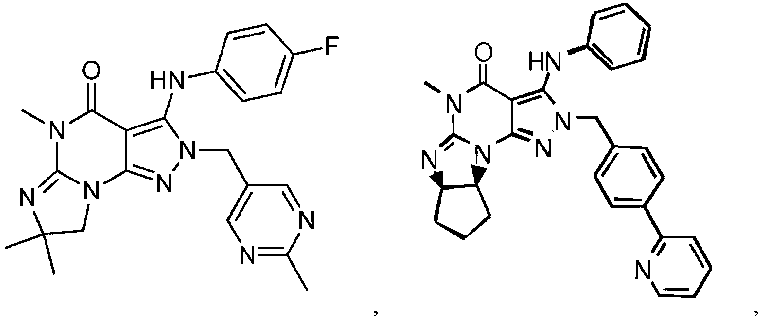 or 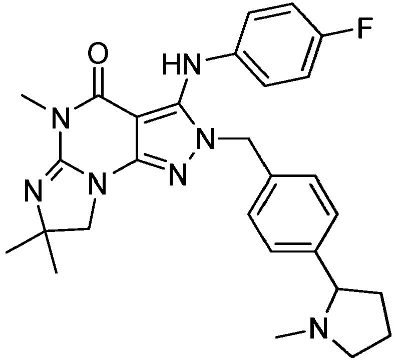 in free or pharmaceutically acceptable salt form.
2.12 Any of the preceding methods, wherein the PDE1 inhibitor administered in step c) is 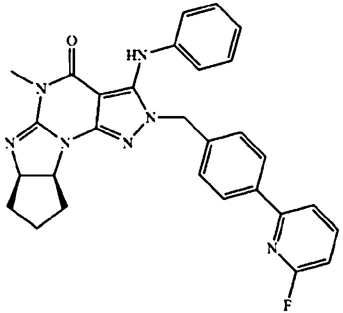 in free or pharmaceutically acceptable salt form.

In yet another embodiment, the present invention provides a compound for use in a method [Method 3] of diagnosing a PDE1-mediated disease, disorder or condition characterized by up-regulation of PDE1 expression in a subject, wherein the PDE1-mediated disease, disorder or condition is a cardiovascular-related disorder, a neurodegenerative disease, a cancer or a tumor, comprising:
a) obtaining a first tissue sample from a patient suspected of having the PDE1-mediated disease, disorder or condition or at risk for the PDE1-mediated disease, disorder or condition;
b) contacting the first tissue sample with an effective amount of a compound according to Formula Ia *et seq.* or II *et seq.* wherein the compound according to Formula Ia *et seq.* or II *et seq* is radiolabeled, e.g., substituted with T (tritium, ³H) at one or more positions in place of H;
c) imaging the first tissue sample with a positron emission tomography device;
d) comparing the results of step c) to a second tissue sample in which PDE1 expression is not up-regulated.

In further embodiments, the invention provides Method 3 as described in the following Methods:
3.1 Method 3, further comprising the step of imaging the second tissue sample with a positron emission tomography device.
3.2 Any of the preceding methods, wherein the second tissue sample is obtained from a subject not suffering from a PDE1-mediated disease, disorder or condition.
3.3 Any of the preceding methods, wherein the first tissue sample and the second tissue sample are both the same type of tissue (e.g., human brain tissue).
3.4 Any of the preceding methods, wherein the first tissue sample is human brain tissue taken from a subject suspected to be suffering from glioblastoma multiforme and the second tissue sample is non-cancerous human brain tissue.
3.5 Any of the preceding methods, wherein if the comparison of step d) shows that PDE1 expression in the first tissue sample is greater than that of the second sample, then the patient is suffering from the PDE1-mediated disease, disorder or condition.
3.7 Any of the preceding Methods, wherein the compound of step a) is 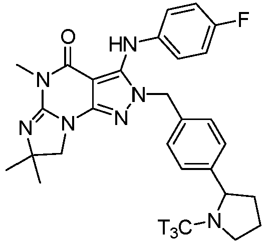 in free or pharmaceutically acceptable salt form.
3.8 Any of the preceding methods, wherein the positron emission tomography is positron emission tomography/computed tomography or single photon emission computed tomography/computed tomography.
3.9 Any of the preceding methods, wherein the PDE1-mediated disease, disorder or condition is a cardiovascular-related disorder, a neurodegenerative disease, cancer or a tumor.
3.10 Any of the preceding methods, wherein the PDE1-mediated disease, disorder or condition is:
   a. a tumor selected from one or more of acoustic neuroma, astrocytoma, chordoma, CNS lymphoma, craniopharyngioma, gliomas (e.g., Brain stem glioma, ependymoma, mixed glioma, optic nerve glioma), subependymoma, medulloblastoma, meningioma, metastatic brain tumors, oligodendroglioma, pituitary tumors, primitive neuroectodermal (PNET), schwannoma, adenomas (e.g., basophilic adenoma, eosinophilic adenoma, chromophobe adenoma, parathyroid adenoma, islet adenoma, fibroadenoma), fibroids (fibrous histiocytoma), fibromas, hemangiomas, lipomas (e.g., angiolipoma, myelolipoma, fibrolipoma, spindle cell lipoma, hibernoma, atypical lipoma), myxoma, osteoma, preleukemias, rhadomyoma, papilloma, seborrheic keratosis, skin adnexal tumors, hepatic adenomas, renal tubular adenoma, bile duct adenoma, transitional cell papilloma, hydatidiform moles, ganglioneuroma, meningoma, neurilemmoma, neurofibroma, C cell hyperplasia, pheochromocytoma, insulinoma, gastrinoma, carcinoids, chemodectoma, paraganglioma, nevus, actinic keratosis, cervical dysplasia, metaplasia (e.g., metaplasia of the lung), leukoplakia, hemangioma, lymphangioma, carcinoma (e.g., squamous cell carcinoma, epidermoid carcinoma, adenocarcinoma, hepatoma, hepatocellular carcinoma, renal cell carcinoma, cholangiocarcinoma, transitional cell carcinoma, embryonal cell carcinoma, parathyroid carcinoma, medullary carcinoma of thyroid, bronchial carcinoid, oat cell carcinoma, islet cell carcinoma, malignant carcinoid,), sarcoma (e.g., fibrosarcoma, myxosarcoma, liposarcoma, chondrosarcoma, osteosarcoma, malignant fibrous histiocytoma, hemangiosarcoma, angiosarcoma, lymphangiosarcoma, leiomyosarcoma, rhabdomyosarcoma, neurofibrosarcoma), blastoma (e.g., medulloblastoma and glioblastoma, types of brain tumor, retinoblastoma, a tumor in the retina of the eye, osteoblastoma, bone tumors, neuroblastoma), germ cell tumor, mesothelioma, malignant skin adnexal tumors, hypernephroma, seminoma, glioma, malignant meningioma, malignant shwannoma, malignant pheochromocytoma, malignant paraganglioma, melanoma, mercell cell neoplasm, cystosarcoma phylloides, or Wilms tumor;
   b. a cancer selected from leukemia (i.e., lymphoctic leukemia or a myelogenous leukemia);
   c. a cardiovascular disorder selected from angina, stroke, renal failure, essential hypertension, pulmonary hypertension, secondary hypertension, isolated systolic hypertension, hypertension associated with diabetes, hypertension associated with atherosclerosis, renovascular hypertension, congestive heart failure, myocardial infarction, angina, stroke and renal failure, hypertension, an inflammatory disease or disorder, Duchenne muscular dystrophy, Becker muscular dystrophy, limb-girdle muscular dystrophy, myotonic dystrophy, and Emery-Dreifuss muscular dystrophy and an connective tissue disease or disorder (e.g., Marfan Syndrome);
   d. a neurodegenerative disease selected from Parkinson's disease, restless leg, tremors, dyskinesias, Huntington's disease, Alzheimer's disease, and drug-induced movement disorders; Mental disorders, including depression, attention deficit disorder, attention deficit hyperactivity disorder, bipolar illness, anxiety, sleep disorders, e.g., narcolepsy, cognitive impairment, dementia, Tourette's syndrome, autism, fragile X syndrome, psychostimulant withdrawal, and drug addiction;
   e. respiratory and inflammatory disorders selected from asthma, chronic obstructive pulmonary disease, and allergic rhinitis, as well as autoimmune and inflammatory diseases;
   f. any disease or condition characterized by low levels of cAMP and/or cGMP (or inhibition of cAMP and/or cGMP signaling pathways) in cells expressing PDE1; and/or
   g. any disease or condition characterized by reduced dopamine D1 receptor signaling activity.
3.11 Any of the preceding Methods, wherein the PDE1-mediated disease, disorder or condition is a glioma, osteosarcoma, melanoma, leukemia, or neuroblastoma.
3.12 Any of the preceding Methods, wherein the PDE1-mediated disease, disorder or condition is a glioma (e.g., ependymoma, astrocytoma, oligodendrogliomas, brain stem glioma, optic nerve glioma, or mixed gliomas, e.g., oligoastrocytomas).
3.13 Any of the preceding Methods, wherein the glioma is an astrocytoma (e.g., glioblastoma multiforme).
3.14 Any of the preceding Methods, wherein the condition is glioblastoma multiforme.
3.15 Any of the preceding Methods, further comprising the step of treating the subject for the PDE1-mediated disease, disorder or condition by administering a pharmaceutically acceptable amount of a PDE1 inhibitor.

The disclosure further provides the use of a PDE1 inhibitor, e.g., any of a Compound of Formula Ia, *et seq.,* or Formula II, *et seq.,* in the manufacture of a medicament for use in any of Methods 1, *et seq.,* Methods 2, *et seq.* or Methods 3 *et seq.*

The invention further provides a PDE1 inhibitor, e.g., any of a Compound of Formula Ia, *et seq.,* or Formula II, *et seq.,* for use in any of Methods 1, *et seq.,* Methods 2, *et seq.* or Methods 3 *et seq.*

The disclosure further provides a pharmaceutical composition comprising a PDE1 inhibitor, e.g., any of a Compound of Formula Ia, *et seq.,* or Formula II, *et seq.,* for use in any of Methods 1, *et seq.,* Methods 2, *et seq.* or Methods 3 *et seq.*

### Methods of Making Compounds of the Disclosure

The PDE1 inhibitors of the Disclosure and their pharmaceutically acceptable salts may be made using the methods as described and exemplified in US 8,273,750, US 2006/0173878, US 8,273,751, US 2010/0273753, US 8,697,710, US 8,664,207, US 8,633,180, US 8,536,159, US 2012/0136013, US 2011/0281832, US 2013/0085123, US 2013/0324565, US 2013/0338124, US 2013/0331363, WO 2012/171016, and WO 2013/192556, and by methods similar thereto and by methods known in the chemical art. Such methods include, but not limited to, those described below. If not commercially available, starting materials for these processes may be made by procedures, which are selected from the chemical art using techniques which are similar or analogous to the synthesis of known compounds.

Various PDE1 inhibitors and starting materials therefor may be prepared using methods described in US 2008-0188492 A1, US 2010-0173878 A1, US 2010-0273754 A1, US 2010-0273753 A1, WO 2010/065153, WO 2010/065151, WO 2010/065151, WO 2010/065149, WO 2010/065147, WO 2010/065152, WO 2011/153129, WO 2011/133224, WO 2011/153135, WO 2011/153136, WO 2011/153138.

The Compounds of the Disclosure include their enantiomers, diastereomers and racemates, as well as their polymorphs, hydrates, solvates and complexes. Some individual compounds within the scope of this disclosure may contain double bonds. Representations of double bonds in this disclosure are meant to include both the E and the Z isomer of the double bond. In addition, some compounds within the scope of this disclosure may contain one or more asymmetric centers. This disclosure includes the use of any of the optically pure stereoisomers as well as any combination of stereoisomers.

It is also intended that the Compounds of the Disclosure encompass their stable and unstable isotopes. Stable isotopes are nonradioactive isotopes which contain one additional neutron compared to the abundant nuclides of the same species (i.e., element). It is expected that the activity of compounds comprising such isotopes would be retained, and such compound would also have utility for measuring pharmacokinetics of the non-isotopic analogs. For example, the hydrogen atom at a certain position on the Compounds of the Disclosure may be replaced with deuterium (a stable isotope which is non-radioactive). Examples of known stable isotopes include, but not limited to, deuterium, 13 C, 15 N, 18 O. Alternatively, unstable isotopes, which are radioactive isotopes which contain additional neutrons compared to the abundant nuclides of the same species (i.e., element), e.g., 123I, 131I, 125I, 11C, 18F, may replace the corresponding abundant species of I, C and F. Another example of useful isotope of the compound of the disclosure is the 11C isotope. Of particular interest in this case is tritium, a radioactive isotope of hydrogen (protium). These radio isotopes are useful for radio-imaging and/or pharmacokinetic studies of the compounds of the disclosure.

Compounds herein referred to as being "radiolabeled" comprise at least one radioactive isotope in place of a nonradioactive isotope in a specified position, e.g. tritium in place of hydrogen, or have a substituent that comprises a radioactive isotope.

Melting points are uncorrected and (dec) indicates decomposition. Temperature are given in degrees Celsius (°C); unless otherwise stated, operations are carried out at room or ambient temperature, that is, at a temperature in the range of 18-25 °C. Chromatography means flash chromatography on silica gel; thin layer chromatography (TLC) is carried out on silica gel plates. NMR data is in the delta values of major diagnostic protons, given in parts per million (ppm) relative to tetramethylsilane (TMS) as an internal standard. Conventional abbreviations for signal shape are used. Coupling constants (J) are given in Hz. For mass spectra (MS), the lowest mass major ion is reported for molecules where isotope splitting results in multiple mass spectral peaks Solvent mixture compositions are given as volume percentages or volume ratios. In cases where the NMR spectra are complex, only diagnostic signals are reported.

The words "treatment" and "treating" are to be understood accordingly as embracing treatment or amelioration of symptoms of disease as well as treatment of the cause of the disease.

For methods of treatment, the word "effective amount" is intended to encompass a therapeutically effective amount to treat a specific disease or disorder.

The term "patient" include human or non-human (i.e., animal) patient. In particular embodiment, the disclosure encompasses both human and nonhuman. In another embodiment, the disclosure encompasses nonhuman. In other embodiment, the term encompasses human.

The term "comprising" as used in this disclosure is intended to be open-ended and does not exclude additional, un-recited elements or method steps.

Dosages employed in practicing the present disclosure will of course vary depending, e.g. on the particular disease or condition to be treated, the particular Compounds of the Disclosure used, the mode of administration, and the therapy desired. Compounds of the Disclosure may be administered by any suitable route, including orally, parenterally, transdermally, or by inhalation, but are preferably administered orally. In general, satisfactory results, e.g. for the treatment of diseases as hereinbefore set forth are indicated to be obtained on oral administration at dosages of the order from about 0.01 to 2.0 mg/kg. In larger mammals, for example humans, an indicated daily dosage for oral administration of both the PDE1 inhibitor will accordingly be in the range of from about 0.50 to 300 mg, conveniently administered once, or in divided doses 2 to 4 times, daily or in sustained release form. Unit dosage forms for oral administration thus for example may comprise from about 0.2 to 150 or 300 mg, e.g. from about 0.2 or 2.0 to 10, 25, 50, 75 100, 150, or 200 mg of a Compound of the Disclosure, together with a pharmaceutically acceptable diluent or carrier therefor.

Compounds of the Disclosure may be administered by any satisfactory route, including orally, parenterally (intravenously, intramuscular or subcutaneous) or transdermally, but are preferably administered orally. In certain embodiments, the Compounds of the Disclosure, e.g., in depot formulation, is preferably administered parenterally, e.g., by injection.

The Compounds of the Disclosure and the Pharmaceutical Compositions of the Disclosure of the Disclosure may be used in combination with one or more additional therapeutic agents, particularly at lower dosages than when the individual agents are used as a monotherapy so as to enhance the therapeutic activities of the combined agents without causing the undesirable side effects commonly occur in conventional monotherapy. Therefore, the Compounds of the Disclosure may be simultaneously, separately, sequentially, or contemporaneously administered with other agents useful in treating disease. In another example, side effects may be reduced or minimized by administering a Compound of the Disclosure in combination with one or more additional therapeutic agents in free or salt form, wherein the dosages of (i) the second therapeutic agent(s) or (ii) both Compound of the Disclosure and the second therapeutic agent, are lower than if the agent/compound are administered as a monotherapy. By way of non-limiting example, such additional therapeutic agents may include ACE inhibitors, Angiotensin II receptor antagonists, calcium channel blockers, etc.

The term "simultaneously" when referring to a therapeutic use means administration of two or more active ingredients at or about the same time by the same route of administration.

The term "separately" when referring to a therapeutic use means administration of two or more active ingredients at or about the same time by different route of administration.

Pharmaceutical compositions comprising Compounds of the Disclosure may be prepared using conventional diluents or excipients and techniques known in the galenic art. Thus, oral dosage forms may include tablets, capsules, solutions, suspensions and the like.

### EXAMPLES

### EXAMPLE 1: Synthesis of novel PDE1 inhibitors

### Synthesis of 3-((4-fluorophenyl)amino)-5,7, 7-trimethyl-2-(4-(1-methylpyrrolidin-2-yl)benzyl)-7,8-dihydro-2H-imidazo[1,2-a]pyrazolo[4,3-e]pyrimidin-4(5H)-one (Compound 1) (FOR REFERENCE ONLY):

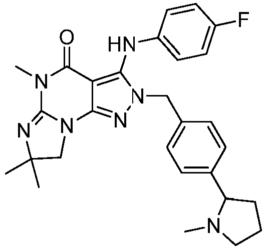

### (a) tert-butyl 2-(4-((5,7,7-trimethyl-4-oxo-4,5,7,8-tetrahydro-2H-imidazo[1,2-a]pyrazolo[4,3-e]pyrimidin-2-yl)methyl)phenyl)pyrrolidine-1-carboxylate

A suspension of 5,7,7-trimethyl-7,8-dihydro-2H-imidazo[1,2-a]pyrazolo[4,3-e]pyrimidin-4(5H)-one (300 mg, 1.37 mmol), tert-butyl 2-(4-(bromomethyl)phenyl)pyrrolidine-1-carboxylate (650 mg, 1.78 mmol) and K₂CO₃ (189 mg, 1.37 mmol) in DMF (3 mL) is stirred at room temperature overnight. Solvent is removed under reduced pressure. The obtained residue is dissolved in ethyl acetate (300mL), sonicated and washed with water (3 × 80 ml). Ethyl acetate is removed and the residue is dried under high vacuum to give 1.07 g of crude product (containing salt), which is used in the next step without further purification. MS (ESI) m/z 479.2 [M+H]+.

### (b) 3-((4-fluorophenyl)amino)-5,7,7-trimethyl-2-(4-(pyrrolidin-2-yl)benzyl)-7,8-dihydro-2H-imidazo[1,2-a]pyrazolo[4,3-e]pyrimidin-4(5H)-one

LiHMDS (1.0 M in hexane, 3.13 mL, 3.13 mmol) is added dropwise to a solution of crude tert-butyl 2-(4-((5,7,7-trimethyl-4-oxo-4,5,7,8-tetrahydro-2H-imidazo[1,2-a]pyrazolo[4,3-e] pyrimidin-2-yl)methyl)phenyl)pyrrolidine-1-carboxylate (1 g, 1.37 mmol) and hexachloroethane (494 mg, 201 mmol) in a mixture of toluene (15 mL) and THF (15 mL) at room temperature. The reaction mixture is stirred at room temperature for 30 minutes, and 4-fluoroanilineis (0.30 mL, 3.13 mmol) is added, followed by LiHMDS (1.0 M in hexane, 3.13 mL, 3.13 mmol). The mixture is stirred at room temperature for additional 10 minutes, and 4-(pyrrolidin-1-yl)pyridine (31 mg, 0.21 mmol) is added. The resulting solution is heated up to 80 °C and stirred at this temperature for 30 minutes. Solvents are removed under reduced pressure and the residue is dissolved in ethyl acetate (200 mL). The ethyl acetate solution is washed with water (3 × 50 mL) and evaporated to dryness under reduced pressure. To the obtained residue is added dichloromethane (5 mL), followed by TFA (5 mL) at room temperature. The mixture is stirred at room temperature overnight. After removal of the solvents, the crude product is loaded on a basic alumina oxide column to give a crude product. This product is further purified with a semi-preparative HPLC system equipped with a reversed-phase C18 column (gradient: 0 - 26% acetonitrile in water containing 0.1% formic acid over 16 min). The title product is obtained as a pale yellow solid (200 mg; three-step overall yield: 30 %). MS (ESI) m/z 488.4 [M+H]+. ¹H NMR (500 MHz, Chloroform-d) δ 8.19 (s, 2H), 7.34 (d, *J =* 7.9 Hz, 2H), 6.97 (d, *J =* 8.0 Hz, 2H), 6.95 - 6.92 (m, 2H), 6.88 - 6.85 (m, 2H), 4.91 (s, 2H), 3.84 (s, 2H), 3.42 - 3.24 (m, 3H), 3.20 (s, 3H), 2.40 - 2.29 (m, 1H), 2.25 - 2.12 (m, 3H), 1.48 (s, 6H).

### (c) 3-((4-fluorophenyl)amino)-5,7,7-trimethyl-2-(4-(1-methylpyrrolidin-2-yl)benzyl)-7,8-dihydro-2H-imidazo[1,2-a]pyrazolo[4,3-e]pyrimidin-4(5H)-one

To a solution of 3-((4-fluorophenyl)amino)-5,7,7-trimethyl-2-(4-(pyrrolidin-2-yl)benzyl)-7,8-dihydro-2H-imidazo[1,2-a]pyrazolo[4,3-e]pyrimidin-4(5H)-one (40 mg, 0.075 mmol) in methanol (0.4 mL) is added 37% formaldehyde (6 1, 0.075mmol). The mixture is stirred at room temperature for 10 min, and sodium cyanoborohydride (5 mg, 0.075 mmol) is added. The resulting mixture is stirred at room temperature for 30 min and then filtered. The filtrate is concentrated and purified with a semi-preparative HPLC system (gradient: 0 - 17% acetonitrile in water containing 0.1% formic acid over 16 min). The title compound is given as a white solid (27 mg, 71% yield). MS (ESI) m/z 502.3. ¹H NMR (500 MHz, Chloroform-d) δ 8.32 (s, 2H), 7.41 (d, *J =* 8.1 Hz, 2H), 7.05 - 6.98 (m, 3H), 6.95 (dd, *J =* 9.0, 8.0 Hz, 2H), 6.89 (dd, *J =* 9.0, 4.6 Hz, 2H), 4.89 (s, 2H), 3.85 (s, 2H), 3.77 (s, 2H), 3.35 (s, 3H), 2.91 (s, 1H), 2.51 (s, 3H), 2.40 - 2.30 (m, 3H), 2.13 - 2.07 (m, 1H), 1.46 (s, 6H).

### Synthesis of 3-((4-fluorophenyl)amino)-5-[3H]methyl-7,7-diimethyl-2-(4-(1-methylpyrrolidin-2-yl)benzyl)-7,8-dihydro-2H-insidazo[1,2-a]pyrazolo[4,3-e]pyrimidin-4(5H)-one (Compound 2)

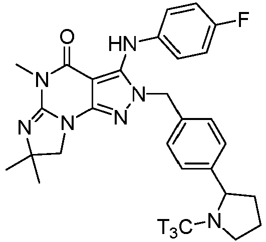

The title compound was synthesized by reacting 3-((4-fluorophenyl)amino)-5,7,7-trimethyl-2-(4-(pyrrolidin-2-yl)benzyl)-7,8-dihydro-2H-imidazo[1,2-a]pyrazolo[4,3-e]pyrimidin-4(5H)-one with [3H]methyl iodide. The obtained crude product was purified by HPLC to give the final compound with >99% radiochemical purity and 58 Ci/mol specific activity. The product identity was confirmed by HPLC co-elution with authentic standard.

Inhibitory constant (Ki) values for Compound 1 were determined against a full panel of phosphodiesterase family members. Compound 1 exhibited potent inhibition for PDE1 enzymes and demonstrated >1000-fold selectivity toward all other PDE families (Table 1). In contrast, potency of Compound 1 for PDE1 A, B and C is roughly equivalent.

**Table 1**

| **PDE Target** | ***K*ᵢ (µM)** | **Ratio PDEx/PDE1A** |
|---|---|---|
| PDE1 (Bovine Brain) | 0.001 | 0.67 |
| PDE1A | 0.0015 | 1 |
| PDE1B | 0.0092 | 5.9 |
| PDE1C | 0.0013 | 0.85 |
| PDE2A | >100 | >64,589 |
| PDE3B | 78.9 | 50,983 |
| PDE4A | 6.6 | 4,263 |
| PDE1D | 2.4 | 1,561 |
| PDE5 | 48.9 | 31,595 |
| PDE6 | 14.1 | 9,122 |
| PDE7B | 13.1 | 8,448 |
| PDE8A | >100 | >64,589 |
| PDE9A | >100 | >64,589 |
| PDE10A | 42.87 | 27,687 |
| PDE11A | 40.70 | 26,289 |

### EXAMPLE 2: Saturation Binding of Novel Radioligand

Whole homogenates of mouse striatum, mouse prefrontal cortex or dog heart were incubated in duplicate at room temperature in a binding buffer containing from nanomolar concentrations of Compound 2. Specific, high affinity binding of Compound 2 to brain and heart was saturable. The *B*ₘₐₓ in mouse striatum was 1252 fmol/mg protein, to mouse cortex 269 fmol/mg protein, and in dog heart 124 fmol/mg protein. The *B*ₘₐₓ value for binding to the striatum was significantly higher than cortex and heart, in agreement with the high expression of PDE1B in this region. The apparent *K*_{d} values for Compound 2 binding was (nM) 2.77, 1.32 and 0.41 for mouse striatum, mouse cortex and dog heart, respectively.

### EXAMPLE 3: Competitive Binding of Compound 2

Various concentrations of a highly potent PDE1 inhibitor were allowed to compete with a fixed concentration (1.5 nM) of Compound 2, a concentration below its *K*_{d} value in striatum. The IC₅₀ values for the competing PDE1 inhibitor were determined from competition assays using Compound 2 binding to mouse striatum and cortex homogenate were 3.45 nM and 0.97 nM respectively. A similar IC₅₀ value (1.11 nM) was determined for the competing PDE1 inhibitor in competition assays using Compound 2 binding to dog heart homogenate. Hill values for displacement of radioligand were -1.20 for striatum, -1.27 in cortex, and -1.03 in dog left ventricle tissues.

Follow up tests were carried out with four additional potent and specific PDE1 inhibitors. These were tested for their potency to inhibit Compound 2 binding to mouse striatum or cortex homogenates. The *K*ᵢ values determined from radioligand competition assay were consistent with the inhibitory constant values from in vitro enzyme assay. *K*ᵢ values for these inhibitors in the binding assay were roughly three times higher than their measured activity in the in vitro PDE1 enzyme activity assay.

### EXAMPLE 4: Calcium Dependency of PDE1 Cellular Activity

Striatal tissue slice preparations were used to further study regulation of PDE1 by intracellular calcium concentration. The striatal slice preparation maintains a portion of the circuitry of the intact striatum including interneurons, nerve terminals from cortical and nigral neuronal inputs, and medium spiny neurons, which account for the majority (95%) of neurons. Depolarization of the cell membrane by increasing external potassium (K+) concentration is a well-established method of stimulating striatal slices, particularly to trigger release of neurotransmitters such as glutamate from the nerve terminals. Under these conditions, both voltage gated calcium channels and NMDA receptors are expected to open, allowing an influx of calcium into the cells. The increase in intracellular calcium can both activate PDE1 in the medium spiny neurons and activate nitric oxide synthase (NOS) in the interneurons via calcium-calmodulin binding. NO released from interneurons activates soluble guanylyl cyclase in the medium spiny neurons triggering an increase in cGMP levels. The global nature of the K+ depolarization triggers the simultaneous stimulation of the medium spiny neurons by neurotransmitter release and depolarization.

When the striatal slice was stimulated with a brief K+ depolarization, preincubation of the slice with 1 µM of a two potent PDE1 inhibitors revealed a significant increase in cGMP levels (Figure 1).

The calcium ionophore ionomycin was used to control intracellular calcium concentrations in the absence of depolarization. Ionomycin (10 µM, in Krebs buffer) increased intracellular calcium, as confirmed by increased calcium-sensitive phosphorylation of Ca2+/calmodulin-dependent protein kinase II (CaMKII), as shown in Figure 2A. Ionomycin-triggered calcium influx alone did not lead to elevated cGMP levels (Figure 2B). However, when PDE1 activity was inhibited in the presence of ionomycin, cGMP levels were significantly increased (p < 0.01, n = 8). Elimination of extracellular calcium by replacing the calcium chloride in the Krebs solution with 3 mM EGTA prevented the elevation in cGMP seen with PDE1 inhibition and ionomycin.

In an additional study, 1321N1 human astrocytoma cells were depleted of Ca2+ by pre-treatment with 10 µM of ionomycin for 15 min in the absence of extracellular Ca2+. The subsequent addition of Ca2+ to the extracellular medium in the presence of the calcium ionophore ionomycin caused a concentration-dependent increase in intracellular Ca2+ concentration as previously reported (8). The elevated Ca2+ level activated the calcium-calmodulin-dependent PDE1 which led to a reduction in the cAMP signal. Figure 1 shows the addition of 3 mM Ca2+ to the extracellular medium caused a substantial inhibition of isoproterenol-evoked cAMP accumulation. This effect was reversed by potent PDE1 inhibition at concentrations in the 0.1 - 1 nanomolar range (Figure 3).

In a further study, binding of Compound 2 (1.5 nM) to mouse cortex was titrated with different concentration of the calcium chelator EGTA to determine the calcium dependency of the ligand binding. EGTA decreased the specific binding of Compound 2 in a concentration-dependent manner with an IC₅₀ value of 7.7 µM (Figure 4). Levels of binding activity remaining in the presence of excess (200uM) EGTA was approximately 5% relative to the binding levels in the absence of EGTA. These results using binding are in reasonable agreement with tests of enzyme activity and establish the absolute calcium dependence of PDE1 inhibitors, i.e., Compound 2, binding to the PDE1 enzyme.

## Claims

1. A radiolabelled compound selected from:
(A) Formula Ia: 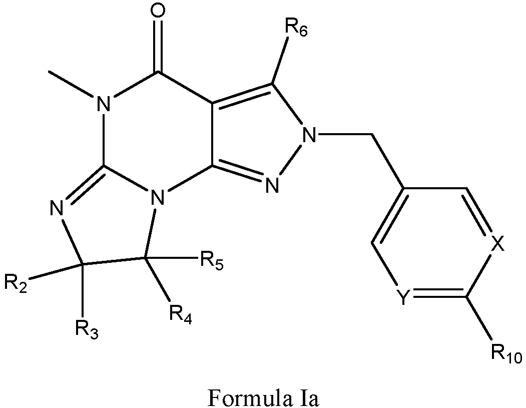 wherein
(i) R₂ and R₅ are independently H or hydroxy and R₃ and R₄ together form a tri- or tetra-methylene bridge [pref. with the carbons carrying R₃ and R₄ having the R and S configuration respectively]; or R₂ and R₃ are each methyl and R₄ and R₅ are each H; or R₂, R₄ and R₅ are H and R₃ is isopropyl [pref. the carbon carrying R₃ having the R configuration];
(ii) R₆ is (optionally halo-substituted) phenylamino, (optionally halo-substituted) benzylamino, C₁₋₄alkyl, or C₁₋₄alkyl sulfide; for example, phenylamino or 4-fluorophenylamino;
(iii) R₁₀ is 1-methylpyrrolidin-2-yl substituted at one or more positions with tritium (T) in place of H; and
(iv) X and Y are independently C or N,
in free, pharmaceutically acceptable salt form, including its enantiomers, diastereoisomers and racemates; and
(B) Formula II: 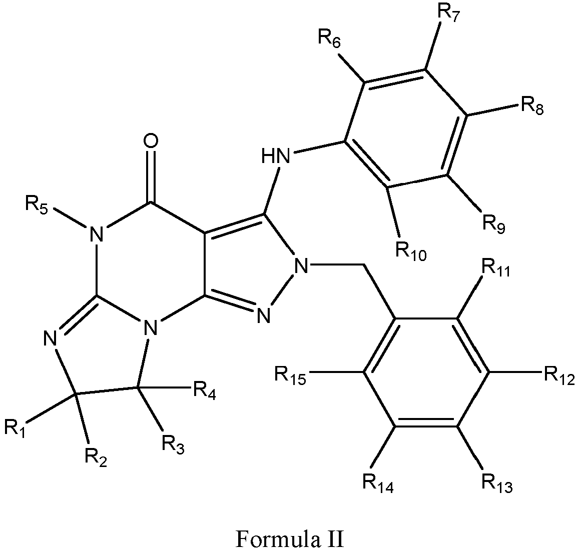 wherein
R₁, R₂ and R₅ are independently selected from optionally T-substituted C₁₋₄alkyl, H or T (tritium, ³H);
R₃, R₄, R₁₁, R₁₂, R₁₄ and R₁₅ are independently selected from H or T (tritium, ³H);
R₆, R₇, R₈, R₉ and R₁₀ are independently selected from halogen (e.g., F), H or T;
R₁₃ is 1-methylpyrrolidin-2-yl substituted at one or more positions with tritium (T) in place of H;
in free, pharmaceutically acceptable salt form, including its enantiomers, diastereoisomers and racemates.

2. A compound according to claim 1, wherein the compound is of Formula II and
(i) R₁, R₂ and R₅ are independently optionally T-substituted C₁₋₄alkyl; or
(ii) R₁, R₂ and R₅ are each optionally T-substituted methyl.

3. A compound according to claim 1, wherein the compound is of Formula II and R₁, R₂ and R₅ are independently T-substituted C₁₋₄alkyl.

4. A compound according to claim 1, wherein the compound is: 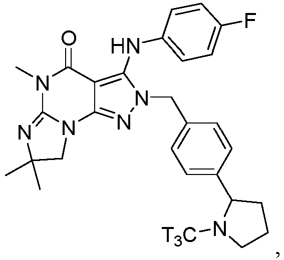 in free, or pharmaceutically acceptable salt form.

5. A compound according to any one of claims 1 to 4 for use in a method of mapping PDE1 activity in tissue and/or organ of interest using positron emission tomography which comprises:
(a) administering an effective amount of a PDE1 tracer compound according to any of claims 1-4, wherein at least one position on the tracer compound is substituted with tritium (T) in place of H;
(b) allowing a period of time sufficient for the tracer to effectively associate with PDE1 in the tissue and/or organ of interest; and
(c) analyzing the tissues and organs of interest using positron emission tomography.

6. The compound for use according to claim 5, wherein the compound according to Formula Ia or II is administered intravenously.

7. A compound according to any one of claims 1 to 4 for use in a method of diagnosing a PDE1-mediated disease, disorder or condition **characterized by** up-regulation of PDE1 expression in a subject, wherein the PDE1-mediated disease, disorder or condition is a cardiovascular-related disorder, a neurodegenerative disease, a cancer or a tumor, comprising
a) obtaining a first tissue sample from a patient suspected of having the PDE1-mediated disease, disorder or condition or at risk for the PDE1-mediated disease, disorder or condition;
b) contacting the first tissue sample with an effective amount of a compound according to any of claims 1-4;
c) imaging the first tissue sample with a positron emission tomography device;
d) comparing the results of step c) to a second tissue sample in which PDE1 expression is not up-regulated.

8. The compound for use according to claim 7, wherein the PDE1-mediated disease, disorder or condition is:
a. a tumor selected from one or more of acoustic neuroma, astrocytoma, chordoma, CNS lymphoma, craniopharyngioma, gliomas (e.g., Brain stem glioma, ependymoma, mixed glioma, optic nerve glioma), subependymoma, medulloblastoma, meningioma, metastatic brain tumors, oligodendroglioma, pituitary tumors, primitive neuroectodermal (PNET), schwannoma, adenomas (e.g., basophilic adenoma, eosinophilic adenoma, chromophobe adenoma, parathyroid adenoma, islet adenoma, fibroadenoma), fibroids (fibrous histiocytoma), fibromas, hemangiomas, lipomas (e.g., angiolipoma, myelolipoma, fibrolipoma, spindle cell lipoma, hibernoma, atypical lipoma), myxoma, osteoma, preleukemias, rhadomyoma, papilloma, seborrheic keratosis, skin adnexal tumors, hepatic adenomas, renal tubular adenoma, bile duct adenoma, transitional cell papilloma, hydatidiform moles, ganglioneuroma, meningoma, neurilemmoma, neurofibroma, C cell hyperplasia, pheochromocytoma, insulinoma, gastrinoma, carcinoids, chemodectoma, paraganglioma, nevus, actinic keratosis, cervical dysplasia, metaplasia (e.g., metaplasia of the lung), leukoplakia, hemangioma, lymphangioma, carcinoma (e.g., squamous cell carcinoma, epidermoid carcinoma, adenocarcinoma, hepatoma, hepatocellular carcinoma, renal cell carcinoma, cholangiocarcinoma, transitional cell carcinoma, embryonal cell carcinoma, parathyroid carcinoma, medullary carcinoma of thyroid, bronchial carcinoid, oat cell carcinoma, islet cell carcinoma, malignant carcinoid,), sarcoma (e.g., fibrosarcoma, myxosarcoma, liposarcoma, chondrosarcoma, osteosarcoma, malignant fibrous histiocytoma, hemangiosarcoma, angiosarcoma, lymphangiosarcoma, leiomyosarcoma, rhabdomyosarcoma, neurofibrosarcoma), blastoma (e.g., medulloblastoma and glioblastoma, types of brain tumor, retinoblastoma, a tumor in the retina of the eye, osteoblastoma, bone tumors, neuroblastoma), germ cell tumor, mesothelioma, malignant skin adnexal tumors, hypernephroma, seminoma, glioma, malignant meningioma, malignant shwannoma, malignant pheochromocytoma, malignant paraganglioma, melanoma, mercell cell neoplasm, cystosarcoma phylloides, or Wilms tumor;
b. a cancer selected from leukemia (i.e., lymphoctic leukemia or a myelogenous leukemia);
c. a cardiovascular disorder selected from angina, stroke, renal failure, essential hypertension, pulmonary hypertension, secondary hypertension, isolated systolic hypertension, hypertension associated with diabetes, hypertension associated with atherosclerosis, renovascular hypertension, congestive heart failure, myocardial infarction, angina, stroke and renal failure, hypertension, an inflammatory disease or disorder, Duchenne muscular dystrophy, Becker muscular dystrophy, limb-girdle muscular dystrophy, myotonic dystrophy, and Emery-Dreifuss muscular dystrophy and an connective tissue disease or disorder (e.g., Marfan Syndrome);
d. a neurodegenerative disease selected from Parkinson's disease, restless leg, tremors, dyskinesias, Huntington's disease, Alzheimer's disease, and drug-induced movement disorders; Mental disorders, including depression, attention deficit disorder, attention deficit hyperactivity disorder, bipolar illness, anxiety, sleep disorders, e.g., narcolepsy, cognitive impairment, dementia, Tourette's syndrome, autism, fragile X syndrome, psychostimulant withdrawal, and drug addiction;
e. respiratory and inflammatory disorders selected from asthma, chronic obstructive pulmonary disease, and allergic rhinitis, as well as autoimmune and inflammatory diseases;
f. any disease or condition **characterized by** low levels of cAMP and/or cGMP (or inhibition of cAMP and/or cGMP signaling pathways) in cells expressing PDE1; and/or
g. any disease or condition **characterized by** reduced dopamine D1 receptor signaling activity.

## Patentansprüche

1. Radioaktiv markierte Verbindung, ausgewählt aus:
(A) Formel Ia: 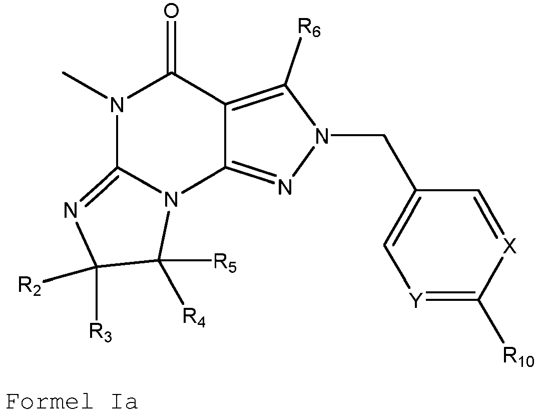 wobei
(i) R₂ und R₅ unabhängig voneinander für H oder Hydroxy stehen und R₃ und R₄ zusammen eine Tri- oder Tetramethylenbrücke bilden [wobei die R₃ und R₄ tragenden Kohlenstoffe vorzugsweise die R- bzw. S-Konfiguration aufweisen] oder R₂ und R₃ jeweils für Methyl stehen und R₄ und R₅ jeweils für H stehen oder R₂, R₄ und R₅ für H stehen und R₃ für Isopropyl steht [wobei der R₃ tragende Kohlenstoff vorzugsweise die R-Konfiguration aufweist],
(ii) R₆ für (gegebenenfalls halogensubstituiertes) Phenylamino, (gegebenenfalls halogensubstituiertes) Benzylamino, C₁₋₄-Alkyl oder C₁₋₄-Alkylsulfid, zum Beispiel Phenylamino oder 4-Fluorphenylamino, steht,
(iii) R₁₀ für 1-Methylpyrrolidin-2-yl steht, das an einer oder mehreren Positionen durch Tritium (T) anstelle von H substituiert ist, und
(iv) X und Y unabhängig voneinander für C oder N stehen, in freier, pharmazeutisch unbedenklicher Salzform, einschließlich ihrer Enantiomere, Diastereoisomere und Racemate, und
(B) Formel II: 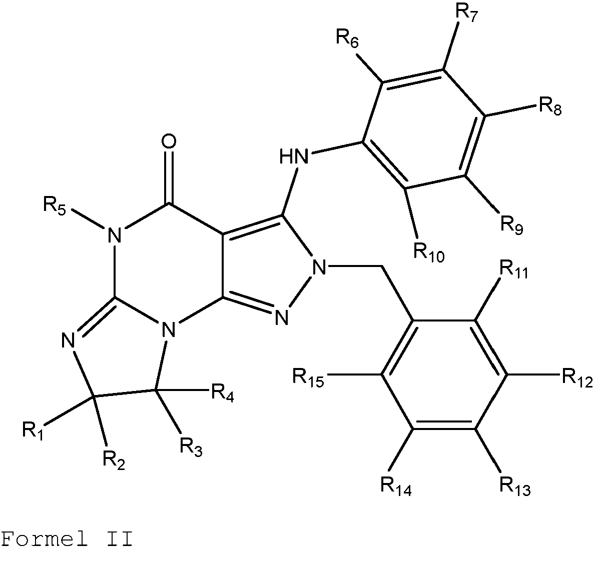 wobei
R₁, R₂ und R₅ unabhängig voneinander aus gegebenenfalls T-substituiertem C₁₋₄-Alkyl, H oder T (Tritium, ³H) ausgewählt sind,
R₃, R₄, R₁₁, R₁₂, R₁₄ und R₁₅ unabhängig voneinander aus H oder T (Tritium, ³H) ausgewählt sind,
R₆, R₇, R₈, R₉ und R₁₀ unabhängig voneinander aus Halogen (z. B. F), H oder T ausgewählt sind,
R₁₃ für 1-Methylpyrrolidin-2-yl steht, das an einer oder mehreren Positionen durch Tritium (T) anstelle von H substituiert ist,
in freier, pharmazeutisch unbedenklicher Salzform, einschließlich ihrer Enantiomere, Diastereoisomere und Racemate.

2. Verbindung nach Anspruch 1, wobei die Verbindung die Formel II aufweist und
(i) R₁, R₂ und R₅ unabhängig voneinander für gegebenenfalls T-substituiertes C₁₋₄-Alkyl stehen oder
(ii) R₁, R₂ und R₅ jeweils für gegebenenfalls T-substituiertes Methyl stehen.

3. Verbindung nach Anspruch 1, wobei die Verbindung die Formel II aufweist und R₁, R₂ und R₅ unabhängig voneinander für T-substituiertes C₁₋₄-Alkyl stehen.

4. Verbindung nach Anspruch 1, wobei die Verbindung die folgende ist: 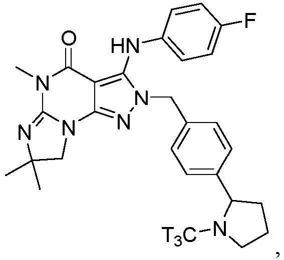 in freier Form oder in Form eines pharmazeutisch unbedenklichen Salzes.

5. Verbindung nach einem der Ansprüche 1 bis 4 zur Verwendung in einem Verfahren zur Abbildung der PDE1-Aktivität in einem Gewebe und/oder Organ von Interesse unter Verwendung von Positronenemissionstomographie, das Folgendes umfasst:
(a) die Verabreichung einer wirksamen Menge einer PDE1-Tracerverbindung nach einem der Ansprüche 1-4, wobei mindestens eine Position an der Tracerverbindung durch Tritium (T) anstelle von H substituiert ist,
(b) Einwirkenlassen einer Zeitspanne, die ausreicht, damit sich der Tracer effektiv an PDE1 im Gewebe und/oder Organ von Interesse binden kann, und
(c) die Analyse der Gewebe und Organe mittels Positronenemissionstomographie.

6. Verbindung zur Verwendung nach Anspruch 5, wobei die Verbindung gemäß Formel Ia oder II intravenös verabreicht wird.

7. Verbindung nach einem der Ansprüche 1 bis 4 zur Verwendung in einem Verfahren zum Diagnostizieren einer PDE1-vermittelten Erkrankung, einer solchen Störung oder eines solchen Leidens, die/das durch eine Hochregulation der PDE1-Expression bei einem Subjekt gekennzeichnet ist, wobei die/das PDE1-vermittelte Erkrankung, Störung oder Leiden eine Herz-Kreislauf-Erkrankung, eine neurodegenerative Erkrankung, eine Krebserkrankung oder ein Tumor ist, umfassend
a) den Erhalt einer ersten Gewebeprobe von einem Patienten, bei dem der Verdacht besteht, dass er die PDE1-vermittelte Erkrankung, eine solche Störung oder ein solches Leiden hat oder bei dem ein Risiko für die PDE1-vermittelte Erkrankung, eine solche Störung oder ein solches Leiden besteht,
b) das Inkontaktbringen der ersten Gewebeprobe mit einer wirksamen Menge einer Verbindung nach einem der Ansprüche 1-4,
c) die bildliche Darstellung der ersten Gewebeprobe mit einer Positronenemissionstomographievorrichtung,
d) den Vergleich der Ergebnisse von Schritt c) mit einer zweiten Gewebeprobe, in der die PDE1-Expression nicht hochreguliert ist.

8. Verbindung zur Verwendung nach Anspruch 7, wobei es sich bei der durch PDE1 vermittelten Erkrankung oder Störung um eine der folgenden handelt:
a. einen Tumor, ausgewählt aus einem oder mehreren der folgenden: Akustikusneurinom, Astrozytom, Chordom, ZNS-Lymphom, Kraniopharyngeom, Gliomen (z. B. Hirnstammgliom, Ependymom, gemischtes Gliom, Sehnervgliom), Subependymom, Medulloblastom, Meningeom, metastatische Hirntumoren, Oligodendrogliom, Hypophysentumoren, primitiven neuroektodermalen Tumoren (PNET), Schwannom, Adenomen (z. B. basophiles Adenom, eosinophiles Adenom, chromophobem Adenom, Nebenschilddrüsenadenom, Inseladenom, Fibroadenom), Fibroiden (fibrozes Histiozytom), Fibromen, Hämangiomen, Lipomen (z. B. Angiolipom, Myelolipom, Fibrolipom, spindelzelliges Lipom, Hibernom, atypisches Lipom), Myxom, Osteom, Präleukämien, Rhabdomyom, Papillom, seborrhoischer Keratose, Hautanhangstumoren, Leberadenomen, renalem tubulärem Adenom, Gallengangsadenom, Übergangszellpapillom, Blasenmolen, Ganglioneurom, Meningeom, Neurilemmom, Neurofibrom, C-Zell-Hyperplasie, Phäochromozytom, Insulinom, Gastrinom, Karzinoiden, Chemodektom, Paragangliom, Naevus, aktinischer Keratose, zervikaler Dysplasie, Metaplasie (z. B. Metaplasie der Lunge), Leukoplakie, Hämangiom, Lymphangiom, Karzinom (z. B. Plattenepithelkarzinom, epidermoidem Karzinom, Adenokarzinom, Hepatom, hepatozellulärem Karzinom, Nierenzellkarzinom, Cholangiokarzinom, Übergangszellkarzinom, embryonalem Karzinom, Nebenschilddrüsenkarzinom, medullärem Schilddrüsenkarzinom, Bronchialkarzinoid, Haferzellkarzinom, Inselzellkarzinom, malignem Karzinoid), Sarkom (z. B. Fibrosarkom, Myxosarkom, Liposarkom, Chondrosarkom, Osteosarkom, malignes fibröses Histiozytom, Hämangiosarkom, Angiosarkom, Lymphangiosarkom, Leiomyosarkom, Rhabdomyosarkom, Neurofibrosarkom), Blastomen (z. B. Medulloblastom und Glioblastom, Arten von Hirntumoren, Retinoblastom, ein Tumor in der Netzhaut des Auges, Osteoblastom, Knochentumoren, Neuroblastom), Keimzelltumor, Mesotheliom, malignen Hautanhangstumoren, Hypernephrom, Seminom, Gliom, malignem Meningeom, malignem Schwannom, malignem Phäochromozytom, malignem Paragangliom, Melanom, Merkelzellkarzinom, Cystosarkom phylloides oder Wilms-Tumor;
b. eine Krebserkrankung, ausgewählt aus Leukämie (d. h. lymphatische Leukämie oder eine myelogene Leukämie);
c. eine Herz-Kreislauf-Erkrankung, ausgewählt aus Angina pectoris, Schlaganfall, Nierenversagen, essenzieller Hypertonie, pulmonaler Hypertonie, sekundärer Hypertonie, isolierter systolischer Hypertonie, mit Diabetes assoziierter Hypertonie, mit Atherosklerose assoziierter Hypertonie, renovaskulärer Hypertonie, Herzinsuffizienz, Myokardinfarkt, Angina pectoris, Schlaganfall und Nierenversagen, Hypertonie, einer entzündlichen Erkrankung oder Störung, Duchenne-Muskeldystrophie, Becker-Muskeldystrophie, Gliedergürtel-Muskeldystrophie, myotoner Dystrophie und Emery-Dreifuss-Muskeldystrophie sowie einer Bindegewebserkrankung oder -störung (z. B. Marfan-Syndrom);
d. eine neurodegenerative Erkrankung, ausgewählt aus Parkinson-Krankheit, Restless-Legs-Syndrom, Tremor, Dyskinesien, Huntington-Krankheit, Alzheimer-Krankheit und medikamenteninduzierten Bewegungsstörungen; psychischen Störungen, einschließlich Depression, Aufmerksamkeitsdefizitstörung, Aufmerksamkeitsdefizit-Hyperaktivitätsstörung, bipolarer Störung, Angstzuständen, Schlafstörungen, z. B. Narkolepsie, kognitiver Beeinträchtigung, Demenz, Tourette-Syndrom, Autismus, Fragiles-X-Syndrom, Entzug von Psychostimulanzien und Drogenabhängigkeit;
e. Atemwegs- und Entzündungserkrankungen, ausgewählt aus Asthma, chronisch obstruktiver Lungenerkrankung und allergischer Rhinitis, sowie Autoimmun- und Entzündungskrankheiten;
f. jede Erkrankung oder jedes Leiden, die/das durch niedrige cAMP- und/oder cGMP-Spiegel (oder eine Hemmung der cAMP- und/oder cGMP-Signalpfade) in PDE1-exprimierenden Zellen gekennzeichnet ist; und/oder
g. jede Erkrankung oder jedes Leiden, die/das durch reduzierte Dopamin-D1-Rezeptor-Signalaktivität gekennzeichnet ist.

## Revendications

1. Composé radiomarqué choisi parmi :
(A) Formule Ia : 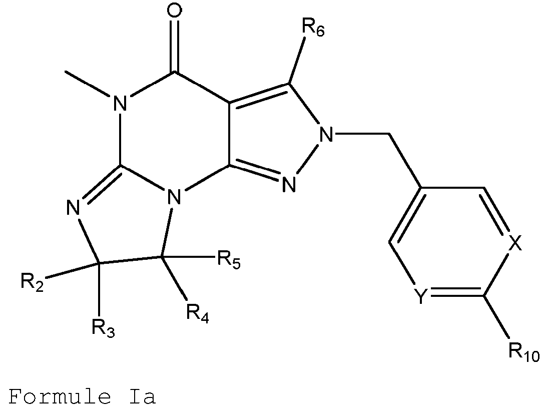 dans laquelle
(i) R₂ et R₅ sont indépendamment H ou hydroxy et R₃ et R₄ forment ensemble un pont tri- ou tétra-méthylène [préf. avec les carbones portant R₃ et R₄ ayant respectivement la configuration R et S] ; ou R₂ et R₃ sont chacun méthyle et R₄ et R₅ sont chacun H ; ou R₂, R₄ et R₅ sont H et R₃ est isopropyle [préf. le carbone portant R₃ ayant la configuration R] ;
(ii) R₆ est phénylamino (éventuellement substitué par halogéno), benzylamino (éventuellement substitué par halogéno), alkyle en C₁₋₄, ou sulfure d'alkyle en C₁₋₄ ; par exemple, phénylamino ou 4-fluorophénylamino ;
(iii) R₁₀ est 1-méthylpyrrolidin-2-yle substitué au niveau d'une ou plusieurs positions par du tritium (T) à la place de H ; et
(iv) X et Y sont indépendamment C ou N,
sous forme libre, de sel pharmaceutiquement acceptable, y compris ses énantiomères, diastéréoisomères et racémiques ; et
(B) Formule II : 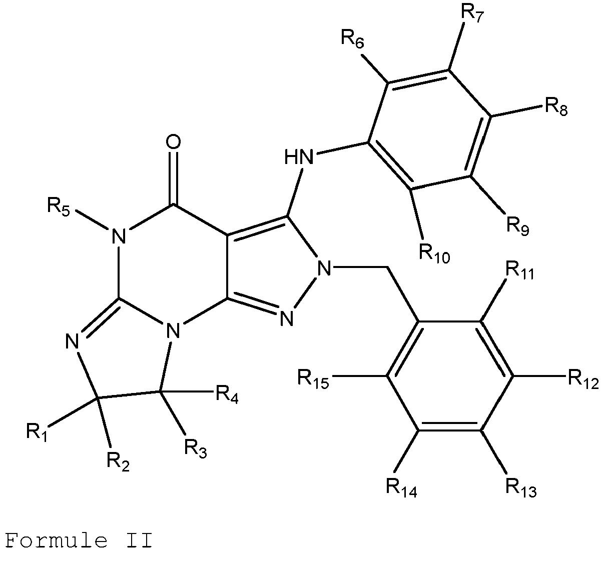 Formule II dans laquelle
R₁, R₂ et R₅ sont choisis indépendamment parmi alkyle en C₁₋₄ éventuellement substitué par T, H ou T (tritium, ³H) ;
R₃, R₄, R₁₁, R₁₂, R₁₄ et R₁₅ sont indépendamment choisis parmi H ou T (tritium, ³H) ;
R₆, R₇, R₈, R₉ et R₁₀ sont indépendamment choisis parmi halogène (par exemple, F), H ou T ;
R₁₃ est 1-méthylpyrrolidin-2-yle substitué au niveau d'une ou plusieurs positions par du tritium (T) à la place de H ;
sous forme libre, de sel pharmaceutiquement acceptable, y compris ses énantiomères, diastéréoisomères et racémiques.

2. Composé selon la revendication 1, dans lequel le composé est de Formule II et
(i) R₁, R₂ et R₅ sont indépendamment alkyle en C₁₋₄ éventuellement substitué par T ; ou
(ii) R₁, R₂ et R₅ sont chacun méthyle éventuellement substitué par T.

3. Composé selon la revendication 1, dans lequel le composé est de Formule II et R₁, R₂ et R₅ sont indépendamment alkyle en C₁₋₄ substitué par T.

4. Composé selon la revendication 1, dans lequel le composé est : 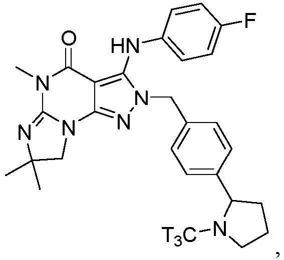 sous forme libre, ou de sel pharmaceutiquement acceptable.

5. Composé selon l'une quelconque des revendications 1 à 4 pour utilisation dans un procédé de cartographie de l'activité de PDE1 dans un tissu et/ou un organe d'intérêt en utilisant une tomographie par émission de positons qui comprend :
(a) l'administration d'une quantité efficace d'un composé traceur de PDE1 selon l'une quelconque des revendications 1 à 4, dans lequel au moins une position sur le composé traceur est substituée par du tritium (T) à la place de H ;
(b) le fait de laisser au traceur une période de temps suffisante pour s'associer efficacement à PDE1 dans le tissu et/ou l'organe d'intérêt ; et
(c) l'analyse des tissus et des organes d'intérêt en utilisant une tomographie par émission de positons.

6. Composé pour utilisation selon la revendication 5, dans lequel le composé selon la Formule Ia ou II est administré par voie intraveineuse.

7. Composé selon l'une quelconque des revendications 1 à 4 pour utilisation dans un procédé de diagnostic d'une maladie, d'un trouble ou d'une affection médié(e) par PDE1 caractérisé(e) par une régulation à la hausse de l'expression de PDE1 chez un sujet, dans lequel la maladie, le trouble ou l'affection médié(e) par PDE1 est un trouble cardiovasculaire, une maladie neurodégénérative, un cancer ou une tumeur, comprenant
a) obtention d'un premier échantillon de tissu chez un patient suspecté d'être atteint de la maladie, du trouble ou de l'affection médié(e) par PDE1 ou présentant un risque pour la maladie, le trouble ou l'affection médié(e) par PDE1 ;
b) mise en contact du premier échantillon de tissu avec une quantité efficace d'un composé selon l'une quelconque des revendications 1 à 4 ;
c) imagerie du premier échantillon de tissu avec un dispositif de tomographie par émission de positons ;
d) comparaison des résultats de l'étape c) à un second échantillon de tissu dans lequel l'expression de PDE1 n'est pas régulée à la hausse.

8. Composé pour utilisation selon la revendication 7, dans lequel la maladie, le trouble ou l'affection médié(e) par PDE1 est :
a. une tumeur choisie parmi l'un ou plusieurs parmi un neurinome acoustique, un astrocytome, un chordome, un lymphome du SNC, un craniopharyngiome, des gliomes (par exemple, un gliome du tronc cérébral, un épendymome, un gliome mixte, un gliome du nerf optique), un subépendymome, un médulloblastome, un méningiome, des tumeurs cérébrales métastatiques, un oligodendrogliome, des tumeurs hypophysaires, une tumeur neuroectodermique primitive (PNET), un schwannome, des adénomes (par exemple, un adénome basophile, un adénome éosinophile, un adénome chromophobe, un adénome parathyroïdien, un adénome insulaire, un fibroadénome), des fibromes (histiocytome fibreux), des fibromes, des hémangiomes, des lipomes (par exemple, un angiolipome, un myélolipome, un fibrolipome, un lipome à cellules fusiformes, un hibernome, un lipome atypique), un myxome, un ostéome, des préleucémies, un rhadomyome, un papillome, une kératose séborrhéique, des tumeurs annexielles cutanées, des adénomes hépatiques, un adénome tubulaire rénal, un adénome des voies biliaires, un papillome à cellules transitionnelles, des nævus hydatiformes, un ganglioneurome, un méningome, un neurilemmome, un neurofibrome, une hyperplasie des cellules C, un phéochromocytome, un insulinome, un gastrinome, des tumeurs carcinoïdes, un chémodecomte, un paragangliome, un nævus, une kératose actinique, une dysplasie cervicale, une métaplasie (par exemple, une métaplasie pulmonaire), une leucoplasie, un hémangiome, un lymphangiome, un carcinome (par exemple, un carcinome épidermoïde, un carcinome épidermoïde, un adénocarcinome, un hépatome, un carcinome hépatocellulaire, un carcinome à cellules rénales, un cholangiocarcinome, un carcinome à cellules transitionnelles, un carcinome embryonnaire, un carcinome parathyroïdien, un carcinome médullaire de la thyroïde, une tumeur carcinoïde bronchique, un carcinome à petites cellules, un carcinome à cellules insulaires, une tumeur carcinoïde maligne), un sarcome (par exemple, un fibrosarcome, un myxosarcome, un liposarcome, un chondrosarcome, un ostéosarcome, un histiocytome fibreux malin, un hémangiosarcome, un angiosarcome, un lymphangiosarcome, un léiomyosarcome, un rhabdomyosarcome, un neurofibrosarcome), un blastome (par exemple, un médulloblastome et un glioblastome, des types de tumeurs cérébrales, un rétinoblastome, une tumeur de la rétine de l'œil, un ostéoblastome, des tumeurs osseuses, un neuroblastome), une tumeur germinale, un mésothéliome, des tumeurs malignes des annexes cutanées, un hypernéphrome, un séminome, un gliome, un méningiome malin, un schwannome malin, un phéochromocytome malin, un paragangliome malin, un mélanome, une néoplasie à cellules de Merkel, un cystosarcome phylloïde ou une tumeur de Wilms ;
b. un cancer choisi parmi une leucémie (c'est-à-dire, une leucémie lymphocytaire ou une leucémie myélogène) ;
c. un trouble cardiovasculaire choisi parmi une angine de poitrine, un accident vasculaire cérébral, une insuffisance rénale, une hypertension essentielle, une hypertension pulmonaire, une hypertension secondaire, une hypertension systolique isolée, une hypertension associée au diabète, une hypertension associée à l'athérosclérose, une hypertension rénovasculaire, une insuffisance cardiaque congestive, un infarctus du myocarde, une angine de poitrine, un accident vasculaire cérébral et une insuffisance rénale, une hypertension, une maladie ou un trouble inflammatoire, une dystrophie musculaire de Duchenne, une dystrophie musculaire de Becker, une dystrophie musculaire des ceintures, une dystrophie myotonique et une dystrophie musculaire d'Emery-Dreifuss et une maladie ou un trouble du tissu conjonctif (par exemple, le syndrome de Marfan) ;
d. une maladie neurodégénérative choisie parmi la maladie de Parkinson, le syndrome des jambes sans repos, les tremblements, les dyskinésies, la maladie de Huntington, la maladie d'Alzheimer et les troubles du mouvement induits par les médicaments ; les troubles mentaux, notamment la dépression, le trouble déficitaire de l'attention, le trouble déficitaire de l'attention avec hyperactivité, la maladie bipolaire, l'anxiété, les troubles du sommeil, par exemple la narcolepsie, les troubles cognitifs, la démence, le syndrome de Tourette, l'autisme, le syndrome de l'X fragile, le sevrage des psychostimulants et la toxicomanie ;
e. les troubles respiratoires et inflammatoires choisis parmi l'asthme, la bronchopneumopathie chronique obstructive et la rhinite allergique, ainsi que les maladies auto-immunes et inflammatoires ;
f. toute maladie ou affection **caractérisée par** de faibles taux de cAMP et/ou cGMP (ou l'inhibition des voies de signalisation de cAMP et/ou cGMP) dans des cellules exprimant PDE1 ; et/ou
g. toute maladie ou affection **caractérisée par** une activité de signalisation du récepteur D1 de dopamine réduite.
